(19) Europäisches Patentamt / European Patent Office / Office européen des brevets

(11) **EP 2 125 851 B1**

(12) **EUROPEAN PATENT SPECIFICATION**

(45) Date of publication and mention of the grant of the patent: **23.06.2010 Bulletin 2010/25**

(21) Application number: **08719365.2**

(22) Date of filing: **04.03.2008**

(51) Int Cl.:
***C07H 17/08*** *(2006.01)* ***A61K 31/7048*** *(2006.01)*
***A61P 31/04*** *(2006.01)*

(86) International application number:
**PCT/IB2008/000715**

(87) International publication number:
**WO 2008/110918 (18.09.2008 Gazette 2008/38)**

(54) **ERYTHROMYCIN-BASED MACROLIDES**

MAKROLIDE AUF ERYTHROMYCINBASIS

MACROLIDES À BASE D'ERYTHROMYCINE

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **13.03.2007 US 894475 P**
**24.01.2008 US 23370 P**

(43) Date of publication of application:
**02.12.2009 Bulletin 2009/49**

(73) Proprietor: **Pfizer Products Inc.**
**Groton, CT 06340 (US)**

(72) Inventors:
- **BUZON, Sr., Richard, Allen**
  **Groton, CT 06340 (US)**
- **FLANAGAN, Mark, Edward**
  **Groton, CT 06340 (US)**
- **LI, Zhengong, Bryan**
  **Groton, CT 06340 (US)**
- **MAGEE, Thomas, Victor**
  **Groton, CT 06340 (US)**
- **NOE, Mark, Carl**
  **Groton, CT 06340 (US)**
- **REILLY, Usa, Datta**
  **Groton, CT 06340 (US)**
- **WIDLICKA, Daniel, William**
  **Groton, CT 06340 (US)**

(74) Representative: **Kingsbury, Oliver William**
**Pfizer Limited**
**European Patent Department**
**Ramsgate Road**
**Sandwich**
**Kent CT13 9NJ (GB)**

(56) References cited:
**WO-A-2006/067589**

- **WU Y-J ET AL: "RECENT DEVELOPMENTS ON KETOLIDES AND MACROLIDES" CURRENT MEDICINAL CHEMISTRY, BENTHAM SCIENCE PUBLISHERS BV, BE, vol. 8, no. 14, 1 December 2001 (2001-12-01), pages 1727-1758, XP009056485 ISSN: 0929-8673**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 125 851 B1

Printed by Jouve, 75001 PARIS (FR)

## Description

### Field of the Invention

The present invention relates to macrolide compounds and their use as antibacterial and antiprotozoal agents in animals, including humans. The invention also relates to methods of preparing compounds, intermediates useful in preparing compounds, and pharmaceutical compositions containing compounds. The present invention further includes methods of treating and/or preventing disease, *e.g.*, bacterial and/or protozoal infections (or for other indications, *e.g.*, cancer, inflammation, or atherosclerosis), by administering compounds or compositions to subjects in need of such treatment.

### Background of the Invention

Macrolides, including ketolides, are generally known as a class to possess antibacterial activity in many instances. Although not limiting to the present invention, it is believed that the macrolide binds to a subunit of the bacterial ribosome, resulting in protein synthesis inhibition. Thus, at least in general terms, the activity of and mechanism of action of erythromycin, clarithromycin, and other macrolides are known.

Erythromycin and clarithromycin are well-known macrolides. Other erythromycin-based macrolide compounds have been prepared, *e.g.*, by introducing modifications at various positions of erythromycin or clarithromycin, *e.g.*, as in: US4331803; US4474768; US4517359; US5523399; US5527780; US5635485; US5804565; US6020521; US6025350; US6075133; US6162794; US6191118; US6248719; US6291656; US6437151; US6472371; US6555524; US2002/0052328; US2002/0061856; US2002/0061857; US2002/0077302; US2002/0151507; US2002/0156027; US2003/0100518; US2003/0100742; US2003/0199458; US2004/0077557; US2006/0135447; WO99/11651; WO99/21866; WO99/21869; WO99/35157; EP1114826; and Tanikawa et al., J. Med. Chem., 46:2706-2715 (2003). Additional relevant publications are cited herein below. Thus, derivatives can include, *e.g.*, modifications at the C-2, C-3, C-6, C-9, C-10, C-11, C-12, and C-13 erythromycin positions, etc., and corresponding azalide derivatives.

There is a continuing need for new macrolides, such as ketolides, in response to the increasing emergence of resistant organisms, to improve safety, and to improve activity spectrum, among other reasons.

### Summary of the Invention

The present invention relates to certain compounds of Formula (I), their preparation and useful intermediates, pharmaceutical compositions thereof, the use thereof for the manufacture of a medicament for treating and preventing bacterial infections therewith. In many embodiments, the compounds are active and effective against organisms that are resistant to other antibiotics, including other macrolides.

In particular, the present invention relates to a compound of Formula (I):

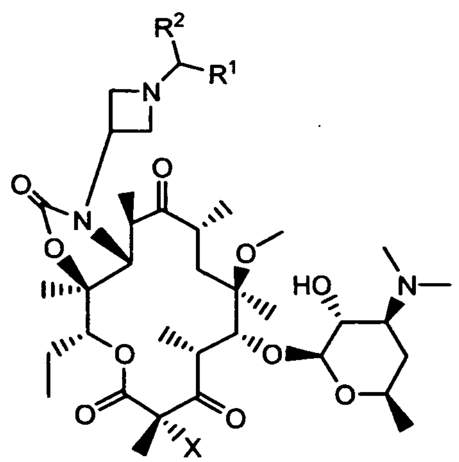

**(I)**

wherein $R^1$ is selected from the group consisting of 3-hydroxy-[1,5]-naphthyridin-4-yl, 3-hydroxy-[1,6]-naphthyridin-4-yl, and 3-hydroxy-[1,7]-naphthyridin-4-yl wherein said 3-hydroxy-[1,5]-naphthyridin-4-yl, 3-hydroxy-[1,6]-naphthyridin-4-yl, and 3-hydroxy-[1,7]-naphthyridin-4-yl are optionally substituted by a group selected from the group consisting of $(C_1-C_3)$alkyl, cyclopropyl, and cyclobutyl;
$R^2$ is selected from the group consisting of hydrogen, methyl, and ethyl; and
X is hydrogen or fluorine;
or a pharmaceutically acceptable salt thereof.

In one embodiment $R^1$ is 3-hydroxy-[1,5]-naphthyridin-4-yl. In another embodiment, $R^1$ is 3-hydroxy-[1,6]-naphthyridin-4-yl. In another embodiment $R^1$ is 3-hydroxy-[1,7]-naphthyridin-4-yl.

In one embodiment, $R^1$ is substituted by methyl. In another embodiment, $R^1$ is substituted by ethyl. In another embodiment, $R^1$ is substituted is substituted by propyl. In another embodiment, $R^1$ is substituted is substituted by cyclopropyl. In another embodiment $R^1$ is substituted by cyclobutyl.

In one embodiment $R^2$ is hydrogen. In another embodiment $R^2$ is methyl. In another embodiment $R^2$ is ethyl.

In one embodiment X is hydrogen. In another embodiment X is fluorine.

In one embodiment, $R^1$ is 3-hydroxy-[1,5]-naphthyridin-4-yl, $R^2$ is hydrogen, and X is hydrogen; or a pharmaceutically acceptable salt thereof.

Examples of specific compounds of Formula (I) include:

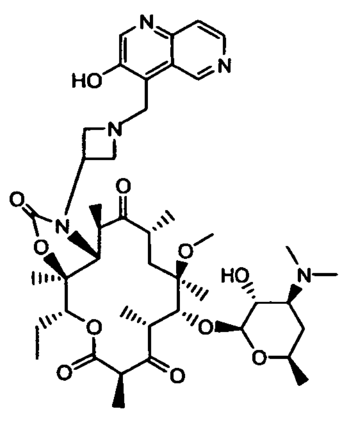 , 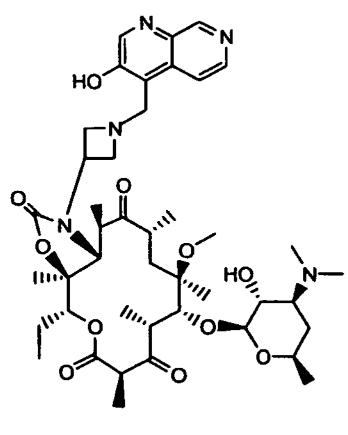 , 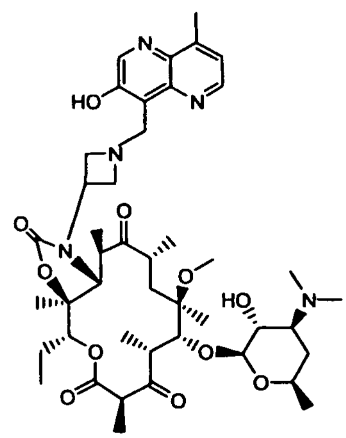 ,

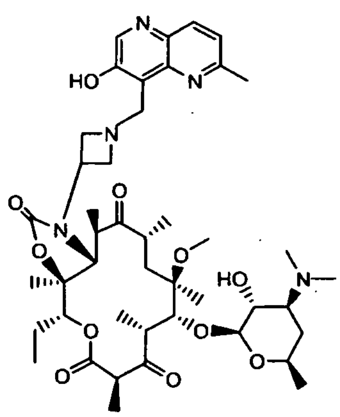 , 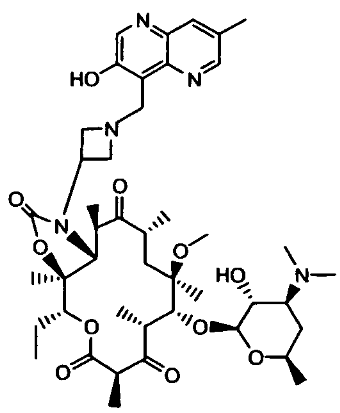 , 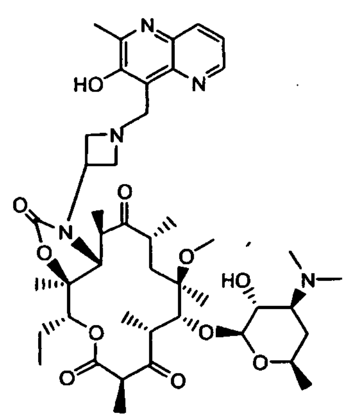 ,

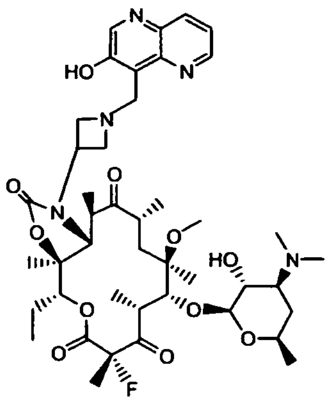
,
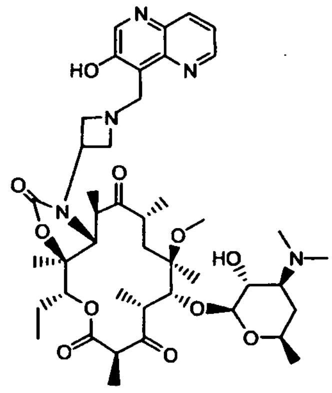
,
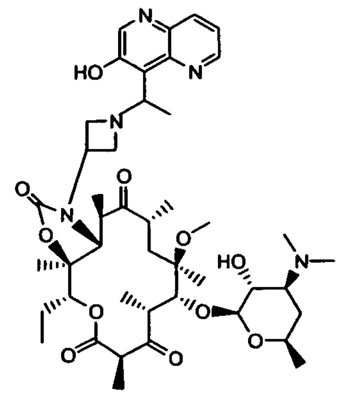
;

and pharmaceutically acceptable salts thereof.

In one embodiment, the compound of Formula (I) is

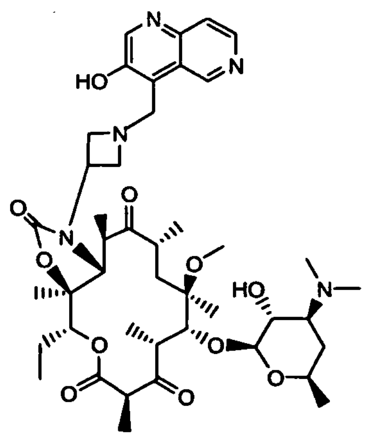

or a pharmaceutically acceptable salt thereof.

In another embodiment, the compound of Formula (I) is

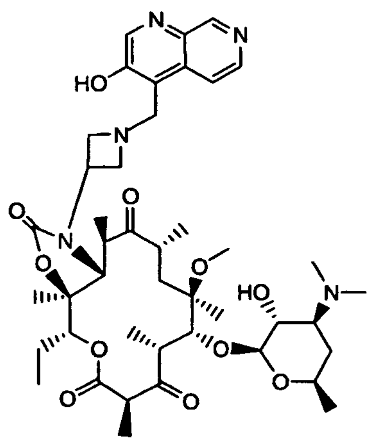

or a pharmaceutically acceptable salt thereof.

In another embodiment, the compound of Formula (I) is

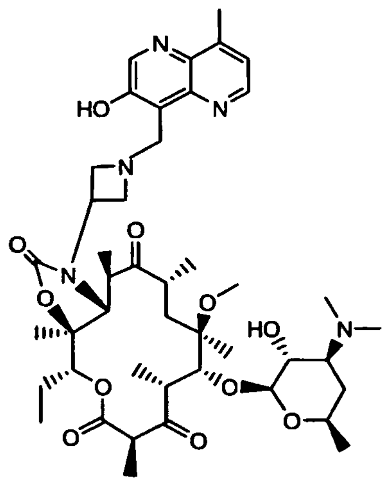

or a pharmaceutically acceptable salt thereof.

In another embodiment, the compound of Formula (I) is

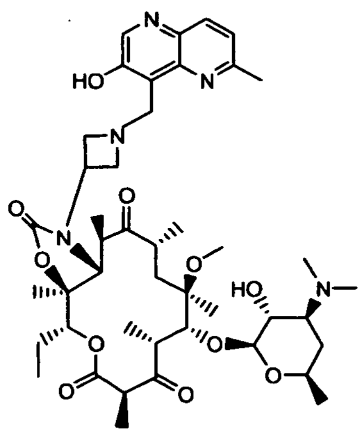

or a pharmaceutically acceptable salt thereof.

In another embodiment, the compound of Formula (I) is

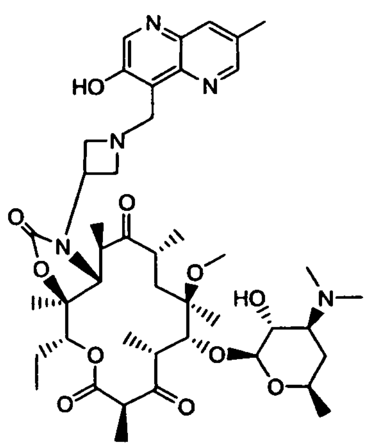

or a pharmaceutically acceptable salt thereof.

In another embodiment, the compound of Formula (I) is

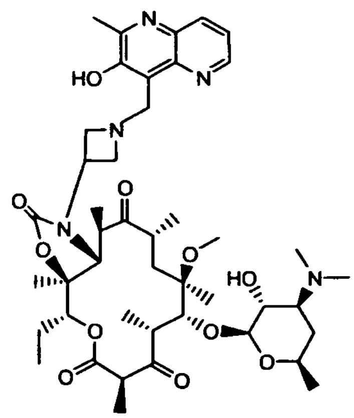

or a pharmaceutically acceptable salt thereof.

In another embodiment, the compound of Formula (I) is

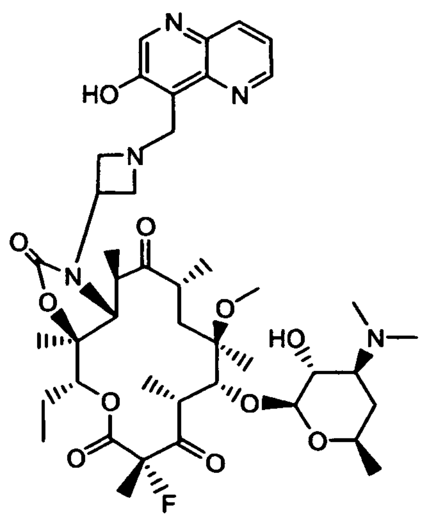

or a pharmaceutically acceptable salt thereof.

In another embodiment, the compound of Formula (I) is

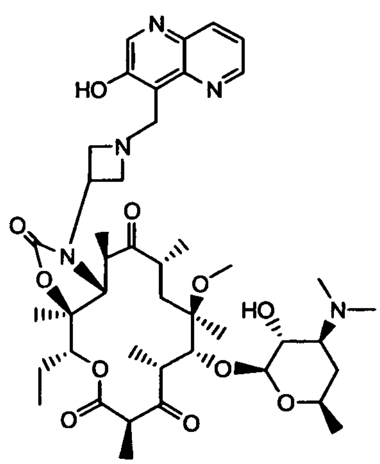

or a pharmaceutically acceptable salt thereof.

In another embodiment, the compound of Formula (I) is

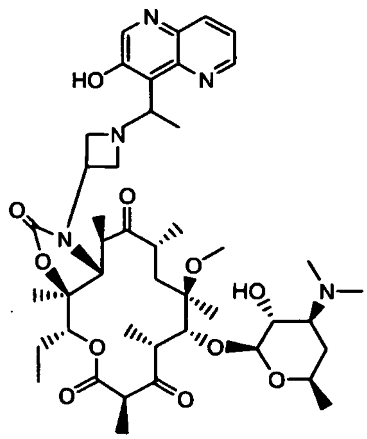

or a pharmaceutically acceptable salt thereof.

In one embodiment of the invention, the pharmaceutically acceptable salt of the compound of Formula (I) is fumarate.

In another embodiment of the invention, the pharmaceutically acceptable salt of the specific structures of a compound of Formula (I) depicted above is fumarate.

The present invention also relates to a pharmaceutical composition comprising the compound of Formula (I), or pharmaceutically acceptable salt thereof, and a pharmaceutically acceptable carrier.

The present invention also relates to the use of a compound of formula (I) in the manufacture of a medicament for the treatment of a bacterial infection in a mammal to be administered to said mammal in an amount that is effective in treating a bacterial infection. In one embodiment, the bacterial infection is selected from the group consisting of community-acquired respiratory tract infections (RTIs), bacterial community-acquired pneumonia (CAP), acute exacerbations of chronic bronchitis (AECB), sinusitis and pharyngitis. In another embodiment the bacterial infection is community-acquired respiratory tract infections (RTIs). In another embodiment the bacterial infection is community-acquired pneumonia (CAP). In another embodiment the bacterial infection is acute exacerbations of chronic bronchitis (AECB). In another embodiment the bacterial infection is sinusitis. In another embodiment the bacterial infection is pharyngitis.

The present invention also relates to the use thereof in the manufacture of a medicament for treating bacterial infection that is resistant to clarithromycin.

The present invention also relates to the use thereof wherein the infection is at least one of *Streptococcus pyogenes* or *Streptococcus pneumoniae* that is resistant to ctarithromycin.

The present invention also relates to the use thereof wherein the infection is caused by at least one of *S. pyogenes, S. pneumoniae, Haemophilus influenzae,* or *Moraxella catarrhalis.*

The invention is also directed to a method of making a compound of Formula (I),

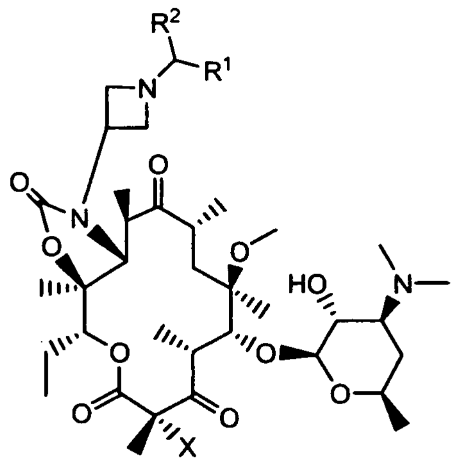

(I)

or pharmaceutically acceptable salt thereof, comprising a first step of reacting a compound of Formula (VIII),

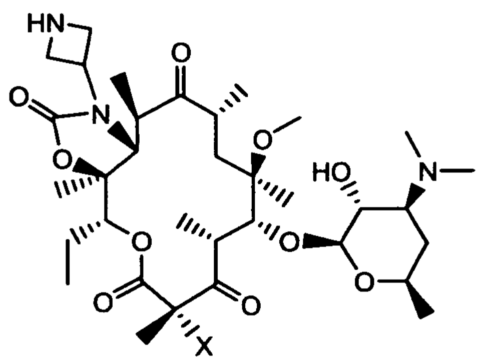

(VIII)

,

with a compound of Formula (IC),

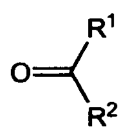

(IC)

in the presence of an acid catalyst and a polar aprotic solvent to form an admixture;
and a second step of treating said admixture with a reducing agent;
wherein $R^1$ is selected from the group consisting of 3-hydroxy-[1,5]-naphthyridin-4-yl, 3-hydroxy-[1,6]-naphthyridin-4-yl, and 3-hydroxy-[1,7]-naphthyridin-4-yl wherein said 3-hydroxy-[1,5]-naphthyridin-4-yl, 3-hydroxy-[1,6]-naphthyridin-4-yl, and 3-hydroxy-[1,7]-naphthyridin-4-yl are optionally substituted by a group selected from the group consisting of $(C_1\text{-}C_3)$alkyl, cyclopropyl, and cyclobutyl;
$R^2$ is selected from the group consisting of hydrogen, methyl, and ethyl;
X is hydrogen or fluorine.

In one embodiment, said acid catalyst is selected from the group consisting of pivalic acid, isobutyric acid, 2,2-dimethylbutanoic acid, and 2-phenylpropanoic acid; said polar aprotic solvent is selected from the group consisting of

tetrahydrofuran (THF), 2-methyl THF, ethyl acetate, isopropyl acetate, acetonitrile, and mixtures thereof; and said reducing agent is selected from the group consisting of sodium triacetoxyborohydride, sodium cyanoborohydride, and hydrogen with a metal catalyst. In one embodiment the metal catalyst is selected from iridium on carbon or platinum on carbon.

In one embodiment, said acid catalyst is pivalic acid and said reducing agent is sodium triacetoxyborohydride.

The invention is also directed to a method of making a compound of Formula (C),

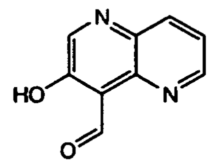

(C)

comprising a first step reacting a compound of Formula (IX),

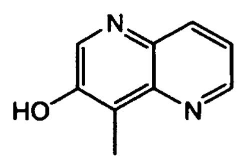

,

(IX)

with a compound of Formula (X),

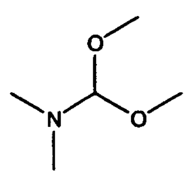

,

(X)

in the presence of a base to make a compound of Formula (XI),

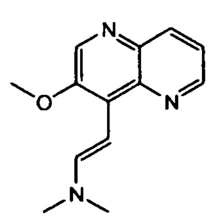

;

(XI)

a second step of reacting a compound of Formula (XI) with sodium periodate in water to form a compound of Formula (XII),

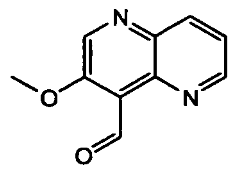

(XII)

and a third step of reacting a compound of Formula (XII) in LiCl or HCl to form a compound of Formula (C).

The present invention includes the use in the manufacture of a medicament of a compound of the invention, including a physiologically acceptable salt or solvate thereof, and including compositions for the treatment or prevention of bacterial or protozoal infections.

The compounds of the invention can also be combined with other active ingredients as desired to attain combination therapy for more than one condition or biological target. For example, the compounds can be combined with other antiinfectives, or agents that increase the efficacy or other properties of antiinfectives, *e.g.*, efflux inhibitors.

The compounds of Formula (I) are useful for treating a patient suffering from a disorder such as, e.g., a bacterial infection.

As used herein the term "patient" refers to a mammal such as, e.g., a human, dog, cat, horse, pig, cow, and the like. In one embodiment, the patient is a human.

Bacterial infections amenable to treatment by compounds of Formula (I), pharmaceutical compositions and methods of the present invention include those caused by a broad range of pathogens, such as, but not limited to, *S. pneumoniae, S. pyogenes, H. influenzae,* and *M. catarrhalis*. In one embodiment the compounds of Formula (I) are useful in treating macrolide-resistant isolates of *S. pneumoniae* and *S. pyogenes.* In other embodiments, the compounds of Formula (I) are useful for treating macrolide-resistant staphylococci and streptococci. In further embodiments, the compounds of Formula (I) are useful in treating *Chlamydia pneumoniae* and *Mycoplasma pneumoniae*. In other embodiments of the present invention the compounds of Formula (I) are useful in treating other streptococci (Lancefield groups B, C, D and G) and *Legionella pneumophila*.

The compounds of Formula (I) may be useful for the treatment of community-acquired respiratory tract infections (RTIs). The compounds of Formula (I) may be useful in the treatment of both sensitive and multi-drug resistant (MDR) respiratory pathogens commonly acquired in the community, including *S. pneumoniae, S. pyogenes, H. influenzae,* and *M. catarrhalis.*

The invention also relates to compositions of the invention which comprise any combination of one or more compounds of Formula (I) and at least one additional ingredient (hereinafter "the compositions of the invention"). In one embodiment, the composition of the invention comprises a therapeutically effective amount of a compound of Formula (I) of the invention.

Non-limiting examples of the at least one additional ingredient include impurities (e.g., intermediates present in the unrefined compounds of Formula (I)), active or pharmaceutical agents as discussed below (e.g., another antibacterial agent), pharmaceutically acceptable excipients, or one or more solvents (e.g., a pharmaceutically acceptable carrier as discussed herein).

Compositions of the invention that are suitable for administration to a patient in need thereof (e.g., a human) are also referred to herein as "pharmaceutical compositions of the invention."

The pharmaceutical composition may be in any form suitable for administration to a patient. For example, the pharmaceutical composition may be in a form suitable for oral administration such as a tablet, capsule, pill, powder, sustained release formulations, solution, and suspension; for parenteral injection as a sterile solution, suspension or emulsion; for topical administration as an ointment or cream; or for rectal administration as a suppository. The pharmaceutical composition may be in unit dosage forms suitable for single administration of precise dosages.

Exemplary parenteral administration forms include solutions or suspensions of active compounds in sterile aqueous solutions, for example, aqueous propylene glycol or dextrose solutions. Such dosage forms can be suitably buffered, if desired.

In one embodiment, the pharmaceutical composition of the invention may be in the form of an oral dosage form. Non-limiting examples of oral dosage forms include, for example, chewable tablets, capsules, pills, lozenges, troches, sachets, powders, syrups, elixirs, solutions and suspensions, and the like, in accordance with standard pharmaceutical practice.

In another embodiment, the pharmaceutical composition of the invention can also be delivered directly to a patient's gastrointestinal tract through a nasogastric tube.

The tablets, pills, capsules, and the like may contain excipients selected from binders such as polyvinylpyrrolidone, hydroxypropylmethyl cellulose (HPMC), hydroxypropylcellulose (HPC), sucrose, gelatin, acacia, gum tragacanth, or com starch; fillers such as microcrystalline cellulose, lactose, sodium citrate, calcium carbonate, dibasic calcium phosphate, glycine and starch; disintegrants such as com starch, potato starch, alginic acid, sodium starch glycolate, croscarmellose sodium and certain complex silicates; lubricants such as magnesium stearate, sodium lauryl sulfate and talc; and sweeteners such as sucrose lactose or saccharin. When a dosage unit form is a capsule, it may contain, in addition to materials of the above type, a liquid carrier such as a fatty oil. Various other materials may be present as coatings or to modify the physical form of the dosage unit. For instance, tablets may be coated with shellac, sugar or both.

In the case of pediatric oral suspensions and sachets, these excipients may comprise suspending aids such as xantham gum or hydroxypropylmethylcellulose, glidants such as colloidal silica, diluents and bulking agents such as silicon dioxide, flavors such as bubble gum, orange, banana, raspberry and golden syrup or mixtures thereof, sweeteners such as aspartame or sugar, and stabilizers such as succinic acid. Powder or granular formulations, such as pediatric suspension formulations and sachets, may be manufactured using techniques which are generally conventional in the field of manufacture of pharmaceutical formulations and in the manufacture of dry formulations for reconstitution into such suspensions. For example a suitable technique is that of mixing dry powdered or granulated ingredients.

The compounds of Formula (I) may, in one embodiment, be used to treat a variety of hospital and community acquired infections such as respiratory tract, surgical, central nervous system, gastrointestinal, genitourinary, gynecological, skin & soft tissue, and ocular infections and community-acquired pneumonia in humans (hereinafter "the infections").

In one embodiment, the infection is selected from the group consisting of acute exacerbation of chronic bronchitis, sinusitis, otitis media, brain abscess, pharyngitis, meningitis, uncomplicated/complicated urinary tract infections, pyelonephritis, hospital-acquired pneumonia, community-acquired pneumonia, surgical prophylaxis, uncomplicated/complicated skin and skin structure infections, intra-abdominal infections, prostatitis, obstetric and gynecological infections, bone and joint infections, diabetic foot, and bacteremia.

In another embodiment, the infection is selected from the group consisting of complicated and uncomplicated urinary tract infections, complicated skin and skin structure infections, diabetic foot infections, community-acquired pneumonia, hospital-acquired pneumonia, intra-abdominal infections, and obstetric and gynecological infections.

In one particular embodiment the infection is otitis media. In another specific embodiment, the infection is complicated skin and skin structure infections.

The minimum amount of the compounds of Formula (I) to be administered is a therapeutically effective amount. The term "therapeutically effective amount" means the amount of compound which prevents the onset of, alleviates the symptoms of, stops the progression of, and/or eliminates a bacterial infection in a mammal, e.g., a human.

Typically, an effective daily dose (i.e., total dosage over about 24 hours) of the compounds of Formula (I) of the invention for adults is about 200 mg to about 6000 mg of compounds of Formula (I); in another embodiment, an effective daily dose is about 800 mg to about 6000 mg; and in another embodiment, an effective daily dose is about 800 mg to about 4000 mg. This daily dose is administered over a period of about one week to about two weeks. In some cases, it may be necessary to use dosages outside these limits.

The compounds of Formula (I) of the invention may be administered in combination with one or more additional medicinal or pharmaceutical agents ("the additional active agent"). Such use of the compounds of Formula (I) in combination with an additional active agent may be for simultaneous, separate or sequential use.

In one embodiment, the additional active agent is an antibacterial agent. Non-limiting examples of useful antibacterial agents include: β-lactams such as penicillins (e.g., amoxicillin and ampicillin), cephalosporins (e.g., cefipime, cefditoren pivoxil (Spectracef®), cephalothin, cefaclor or cefixime;□-lactamase inhibitors and □-lactam/□-lactamase inhibitor combinations such as sulbactam, clavulanic acid, tazobactam and piperacillin-tazobactam (Zosyn®); macrolides such as azithromycin, erythromycin, or clarithromycin; and fluoroquinolones such as norfloxacin, ciprofloxacin, levofloxacin (Levaquin®), moxifloxacin (Avelox®), or enoxacin. Other non-limiting examples of additional antibacterial agents can be found in Walsh and Wright, Chemical Reviews 105(2):391-394 (2005); and Bush et al., Current Opinion in Microbiology 7:466-476 (2004).

In as much as macrolide antibiotics have shown anti-inflammatory effects (See, for example, Scaglione et al., Journal of Antimicrobial Chemotherapy, 41, Supplement B:47-50 (1998) and Ianaro et al., The Journal of Pharmacology and Experimental Therapeutics 292:156-163 (2000)), other potential uses for compounds of Formula (I) may include dampening the immune responses in inflammatory diseases including rheumatoid arthritis. Although the mechanism of action for this anti-inflammatory activity is unclear, recent evidence suggests that agents such as hydroxychloroquine that increase the pH of endosomal compartments have the ability to inhibit some Toll-like receptor (TLR) signaling. It is conceivable that macrolides may also possess this property and as such, could be useful as TLR 7,8, or 9 antagonists (Kyburz et al., Nature Clinical Practice Rheumatology 2:458-459 (2006)).

In one embodiment, the one or more additional active agents, when used, are administered prior to administration of a compound of Formula (I). In another embodiment, the one or more additional active agents, when used, are administered after administration of a compound of Formula (I). In another embodiment, the one or more additional

active agents, when used, are administered at about the same time as administration of a compound of Formula (I).

The additional active agent may be administered by any route useful to administer said additional active agent.

In one embodiment, the one or more additional active agents are present in the pharmaceutical composition of the invention. Accordingly, in another embodiment, the invention relates to a method of treating a patient with a pharmaceutical composition of the invention further comprising one or more additional active agents.

It is to be understood that any section headings and subheadings herein are for the convenience of the reader and are non-limiting. For example, the subject matter in the Summary of the Invention has no special status solely as a result of its placement in that section.

Unless otherwise indicated, the language and terms used in this document are to be given their broadest reasonable interpretation as understood by the relevant skilled artisan. In addition, in descriptions and claims in which the subject matter (*e.g.*, substitution at a given molecular position) is recited as being selected from a group of possibilities, the recitation is specifically intended to include any subset of the recited group. In the case of multiple variable positions or substituents, any combination of group or variable subsets is also contemplated.

Unless otherwise stated, the following abbreviations have the following meaning: "mmol" means "millimoles", "mol" means "moles", "GCMS" means "Gas Chromatographic Mass Spectrometer" (all samples herein were run on an Agilent Technologies 5975 GCMS), "room temperature" means "ambient temperature" "QD" means "once a day", "Ac" means "acetyl", "AUC" means "area under the curve", "Ph" means "phenyl", "Bz" means "benzoyl", "CBZ" means "benzyloxycarbonyl", "CDI" means "carbonyldiimidazole", "DBU" means "1,8-diazabicyclo[5.4.0]undec-7-ene", "DCM" or "$CH_2Cl_2$" means "dichloromethane", "DMAP" means "dimethylaminopyridine", "DMF" means"dimethylformamide", "DMF/DMA" means "N,N-dimethylformamide dimethyl acetal", "DMSO" means "dimethylsulfoxide", "EDC" means "1-(3-dimethylaminoprcpyl)-3-ethylcarbodiimide hydrochloride", "EtOAc" means "ethyl acetate", "EtOH" means "ethanol", "HCl" means "hydrochloric acid", "IPE" means "diisopropyl ether", "MeCN" means "acetonitrile", "MeOH" means "methanol", "MIC" means "minimum inhibitory concentration", "MS" means "Mass Spectrometry" (all samples herein were analyzed either by LCMS-electrospray (gradient elution using acetonitrile, water, formic acid mixtures) or probe APCI methods), "$NaHCO_3$" means "sodium bicarbonate", "$Na_2SO_4$ means "sodium sulfate", "$NH_4OH$" means "concentrated aqueous ammonium hydroxide solution of 28-30%", "NMR" means "nuclear magnetic resonance spectroscopy" (All samples herein were run at 400 MHz on Varian instruments), "TEA" means "triethylamine", and "THF" means "tetrahydrofuran"

As used herein, the term "($C_1$-$C_3$)alkyl" refers to linear or branched (e.g., methyl, ethyl, n-propyl, isopropyl) radicals of 1 to 3 carbon atoms.

As used herein, the term "dihydrochloride" means "approximately two equivalents of HCl."

Unless otherwise noted herein, erythromycin-based macrolide carbons are identified by the numbering convention shown below:

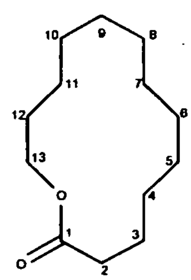

Unless otherwise apparent or indicated, the compounds of the invention and term "compound" in the claims embraces any pharmaceutically acceptable salts or solvates, and any amorphous or crystal forms, or tautomers, whether or not specifically recited in context. Similarly, a recitation is open to any material or composition containing the recited compound (*e.g.*, a composition containing a salt of a racemic mixture of compounds, tautomers, epimers, stereoisomers, impure mixtures, etc.).

The compounds of Formula (I) may exist in unsolvated and solvated forms. Thus, it will be understood that the compounds of the invention also include hydrate and solvate forms as discussed below.

The term "solvent" as it relates to the compositions of the invention includes organic solvents (e.g., methanol, ethanol, isopropanol, ethyl acetate, methylene chloride, and tetrahydrofuran) and water. The one or more solvents may be present in a non-stoichiometric amount, e.g., as a trace impurity, or in sufficient excess to dissolve the compound of the invention. Alternatively, the one or more solvents may be present in a stoichiometric amount, e.g., 0.5:1, 1:1, or 2: 1 molar ratio, based on the amount of compound of the invention.

The term "solvate" is used herein to describe a noncovalent or easily reversible combination between solvent

and solute, or dispersion means and disperse phase. It will be understood that the solvate can be in the form of a solid, slurry (e.g., a suspension or dispersion), or solution. Non-limiting examples of solvents include ethanol, methanol, propanol, acetonitrile, dimethyl ether, diethyl ether, tetrahydrofuan, methylene chloride, and water. The term 'hydrate' is employed when said solvent is water.

A currently accepted classification system for organic hydrates is one that defines isolated site, or channel hydrates - see Polymorphism in Pharmaceutical Solids by K. R. Morris (Ed. H. G. Brittain, Marcel Dekker, 1995). Isolated site hydrates are ones in which the water molecules are isolated from direct contact with each other by intervening organic molecules. In channel hydrates, the water molecules lie in lattice channels where they are next to other water molecules.

When the solvent or water is tightly bound, the complex will have a well-defined stoichiometry independent of humidity. When, however, the solvent or water is weakly bound, as in channel solvates and hygroscopic compounds, the water/solvent content will be dependent on humidity and drying conditions. In such cases, non-stoichiometry will be the norm.

The term "pharmaceutically acceptable salt(s)", as used herein, unless otherwise indicated, includes salts of acidic or basic groups which can be present in the compounds. Compounds that are basic in nature are capable of forming a wide variety of salts with various inorganic and organic acids. The acids that can be used to prepare pharmaceutically acceptable acid addition salts of such basic compounds are those that form non-toxic acid addition salts. The compounds can form, *e.g.*, sulfates, phosphates, citrates, acetates, tosylates, succinates, besylates, mesylates, lactates, and hydrochlorides. Basic salts can be mono or dibasic. In one preferred embodiment, the salt is a fumarate.

The terms "treat," "treatment," and "treating", as used herein in the context of using the compounds of the present invention, unless otherwise indicated, means reversing, alleviating, inhibiting the progress of, or partially or completely preventing the disorder or condition to which such term applies, or one or more symptoms of such disorder or condition. "Preventing" means treating before an infection occurs.

The term "pharmaceutical composition" refers to an active compound in any form suitable for effective administration to a subject, *e.g.*, a mixture of the compound and at least one pharmaceutically acceptable carrier.

The term "pharmaceutically acceptable carrier" refers to a material that can be administered to a subject together with a compound in a pharmaceutical composition. The carrier should not destroy the pharmacological activity of the compound and should be non-toxic when administered in doses sufficient to deliver a therapeutic amount of the compound.

The term "excipient" means an inert material that is combined with the compounds of Formula (I) to produce a pharmaceutical composition or oral drug dosage form. The term "pharmaceutically acceptable excipient" means that the excipient must be compatible with other ingredients of the composition, and not deleterious to the recipient thereof. The pharmaceutically acceptable excipients are chosen on the basis of the intended dosage form.

Compounds of the present invention have asymmetric centers and therefore can exist in different enantiomeric and diastereomeric forms. The invention includes all optical isomers and stereoisomers, and mixtures thereof in all ratios, and to all pharmaceutical compositions and methods of treatment that can employ or contain them. Although specific compounds exemplified in this application can be depicted in a particular stereochemical configuration, compounds having either the opposite stereochemistry at any chiral centers or mixtures thereof are also envisioned. The foregoing can be present as mixtures or enriched in any component to any degree. Where stereochemistry at a position is not specified, such is intended to encompass either configuration or a mixture of any ratio.

Compounds of this invention include pharmaceutically acceptable derivatives or prodrugs thereof. A "pharmaceutically acceptable derivative or prodrug" means any pharmaceutically acceptable salt, ester, salt of an ester or other derivative of a compound that, upon administration to a recipient, is capable of providing (directly or indirectly) a compound of this invention or a metabolite or residue thereof. Particularly favored derivatives and prodrugs of the invention are those that increase the bioavailability of the compounds when such compounds are administered to a patient (*e.g.*, by allowing an orally administered compound to be more effectively absorbed into the blood), enhance delivery of the parent compound to a given biological compartment, increase solubility to allow administration by injection, alter metabolism or alter rate of excretion.

The compounds of Formula (I) may exhibit polymorphism. Polymorphic compounds of Formula (I) may be prepared by crystallization of a compound of the present invention under various conditions. For example, there may be employed various solvents (including water) or different solvent mixtures for recrystallization; crystallization at different temperatures; various modes of cooling ranging from very fast to very slow cooling during crystallization. Polymorphs may also be obtained by heating or melting a compound of the present invention followed by gradual or fast cooling. The presence of polymorphs may be determined by solid probe NMR spectroscopy, IR spectroscopy, differential scanning calorimetry, powder X-ray diffraction or other such techniques.

The present invention includes compounds wherein one or more hydrogen, carbon or other atoms are replaced by different isotopes thereof. Such compounds can be useful as research and diagnostic tools in metabolism pharmacokinetic studies and in binding assays. These isotopically-labeled compounds are identical to those compounds of

Formula (I), but for the fact that one or more atoms are replaced by an atom having an atomic mass or mass number different from the atomic mass or mass number predominantly found in nature. Examples of isotopes that can be incorporated into compounds of the invention include isotopes of hydrogen, carbon, nitrogen, oxygen, and sulfur, such as, but not limited to, $^{2}H$, $^{3}H$, $^{13}C$, $^{14}C$, $^{15}N$, $^{18}O$, $^{17}O$, and $^{35}S$, respectively. The compounds of Formula (I) of the invention containing the aforementioned isotopes and/or other isotopes of these atoms are within the scope of this invention. Certain isotopically-labeled compounds of Formula (I), for example those into which radioactive isotopes such as $^{3}H$ and $^{14}C$ are incorporated, are useful in drug and/or substrate tissue distribution assays. Tritiated, i.e., $^{3}H$, and carbon-14, i.e., $^{14}C$, isotopes are particularly preferred for their ease of preparation and detectability. Further, substitution with isotopes such as deuterium, i.e., $^{2}H$, can afford certain therapeutic advantages resulting from greater metabolic stability, for example increased in vivo half-life or reduced dosage requirements and, hence, may be preferred in some circumstances. Isotopically-labeled compounds of the invention can generally be prepared by carrying out the procedures disclosed in the Schemes and/or in the Examples and described below, by substituting a readily available isotopically-labeled reagent for a non-isotopically-labeled reagent.

The term "protecting group" refers to a suitable chemical group that can be attached to a functional group and removed at a later stage to reveal the intact functional group. Examples of suitable protecting groups for various functional groups are described in T. W. Greene and P. G. M. Wuts, Protective Groups in Organic Synthesis, 2nd Ed., John Wiley and Sons (1991 and later editions); L. Fieser and M. Fieser, Fieser and Fieser's Reagents for Organic Synthesis, John Wiley and Sons (1994); and L. Paquette, ed. Encyclopedia of Reagents for Organic Synthesis, John Wiley and Sons (1995). The term "hydroxy protecting group", as used herein, unless otherwise indicated, includes Ac, Bz, and various hydroxy protecting groups familiar to those skilled in the art, including the groups referred to in Greene.

The examples and preparations provided below further illustrate and exemplify the compounds of the present invention and methods of preparing such compounds. It is to be understood that the scope of the present invention is not limited in any way by the scope of the following examples and preparations.

All patents, patent applications, publications, test methods, literature, and other materials cited above and below herein are hereby incorporated herein by reference in their entireties.

Detailed Description of the Invention

As noted above, in one embodiment, the present invention relates to compounds of Formula (I) and pharmaceutically acceptable salts thereof, as described above. The compounds of Formula (I) are depicted structurally in the Summary of the Invention an elsewhere herein for the convenience of the reader. It is too be understood that compounds of Formula (I) have the same stereochemistry as clarithromycin, wherein the absolute stereochemistry at the C-11 position is (R). An alternative way of structurally depicting a compound of Formula (I) is:

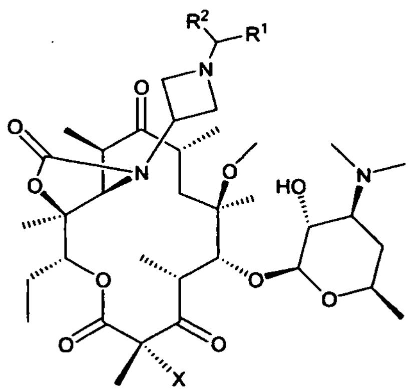

**(I)**

General Preparation Methods

The compounds of the present invention may be prepared according to the descriptions, schemes, and examples herein, which are non-limiting, in combination with the knowledge of the skilled artisan.

Scheme 1

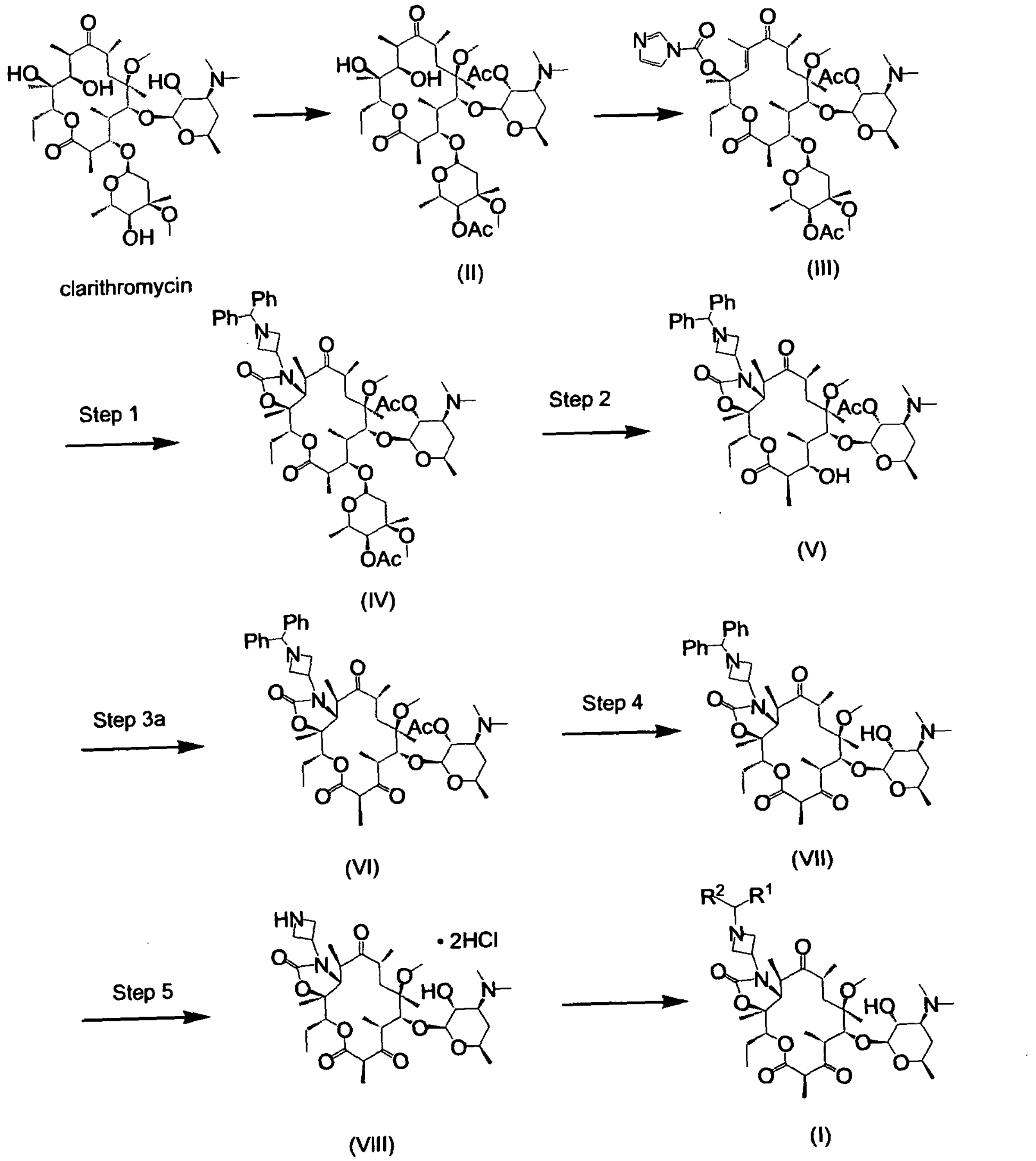


clarithromycin (II) (III) (IV) (V) (VI) (VII) (VIII) (I)

Scheme I illustrates the synthetic scheme used to arrive at a compound of Formula (I). Erythromycin and clarithromycin are known and commercially available, and can be suitable starting materials for preparing compounds of the invention. *See, e.g.*, US 2,653,899; US 2,823,203; and US 4,331,803. Methods well known in the art can be used to protect clarithromycin at 2' and 4" (II) such as by reaction with 2 equivalents of acetic anhydride in a solvent such as DCM, THF, or toluene in the presence of a base such as TEA and in the presence of an activator or catalyst such as DMAP at about room temperature for about 2 hours to 24 hours. 11-Deoxy-2',4"-diacetyl-10,11-didehydrc-12-O-((1H-imidazol-1-yl)-carbonyl)-6-O-methyl-erythromycin A (III) can then be prepared by reaction of (II) with about 3 to 5 equivalents of CDI. See, *e.g.*, Baker et al., J. Org. Chem., 53:2340-2345 (1988). The reaction can be carried out in an aprotic solvent such as MeCN, THF, IPE, DMF, or a mixture thereof in the presence of about 2 to 5 equivalents of a base such as DBU at about 25 to 40°C for about 2 to 12 hours. (III) is reacted with a "liner", such as 1-benzhydryl-azetidin-3-ylamine (see Preparation A below) or other similar compounds described in WO 2007/026207, published March 8, 2007, to make

(IV). The reaction typically employs an amine base such as DBU, Huenig's base, triethylamine, etc., and an aprotic solvent such as acetonitrile, THF, DMF, etc., at a temperature of 25-80°C for 2 to 16 hours. Next, the cladinose moiety at the C-3 position is removed by acidic hydrolysis at 25-40°C to provide (V). The standard condition for this reaction is aqueous HCl in ethanol, however, other conditions may also be employed such as, for example, isopropanol or 1-propanol with aqueous HCl, THF/aqueous HCl, acetone/aqueous HCl, etc. This is followed by oxidation of the alcohol to a ketone to form (VI). This may be accomplished using N-chlorosuccinimide (NCS) and dimethyl sulfide in $CH_2Cl_2$; or other oxidation conditions known to one skilled in the art.

The protecting group Ac is then removed to form (VII) by reaction in methanol at 25°C to reflux temperature. The benzhydryl group is then removed using the catalyst Pd on carbon or $Pd(OH)_2$ on carbon under hydrogen pressure at 20-80°C to form (VIII). The synthesis of (VIII), 3-descladinosyl-11,12-dideoxy-6-O-methyl-3-oxo-12,11-(oxycarbonyl-(azetidin-3-yl)-imino)-erythromycin A dihydrochloride, is described in Preparation B. The final step is accomplished using methods known to one skilled in the art such as a reductive amination with an aldehyde (or an alkylation with an alkyl halide) with the azetidine macrolide to provide a compound of Formula (I). The reductive amination may be carried out in the presence of an acid catalyst, such as, for example, pivalic acid, isobutyric acid, 2,2-dimethylbutanoic acid, 2-phenylpropanoic acid, and other hindered carboxylic acids in a polar aprotic solvent such as THF, 2-methyl THF, ethyl acetate, isopropyl acetate, acetonitrile, etc. The azetidine macrolide and aldehyde (or ketone) are combined, optionally treated with the acid catalyst, and the water generated in the reaction is azeotropically removed. The reaction mixture is then treated with a reducing agent such as sodium triacetoxyborohydride ($NaBH(OAc)_3$), or sodium cyanoborohydride in MeCN or other similar solvents (e.g., THF, EtOAc, etc.) to give the compound of Formula (I). The headpiece can be prepared using methods known to one skilled in the art, using the exemplified 3-hydroxy-[1,5]-naphthyridin-4-carbaldehyde synthesis, described in Preparation C, as a guide. Alternatively, the azetidine macrolide may be coupled directly to a hydroxy-protected headpiece in the same manner as an unprotected headpiece. For example, the pivalate ester of 3-hydroxy-[1,5]-naphthyridin-4-carbaldehyde (the synthesis of which is described in Preparation D) may be used in place of 3-hydroxy-[1,5]-naphthyridin-4-carbaldehyde during the final step of Scheme 1.

## Scheme 2

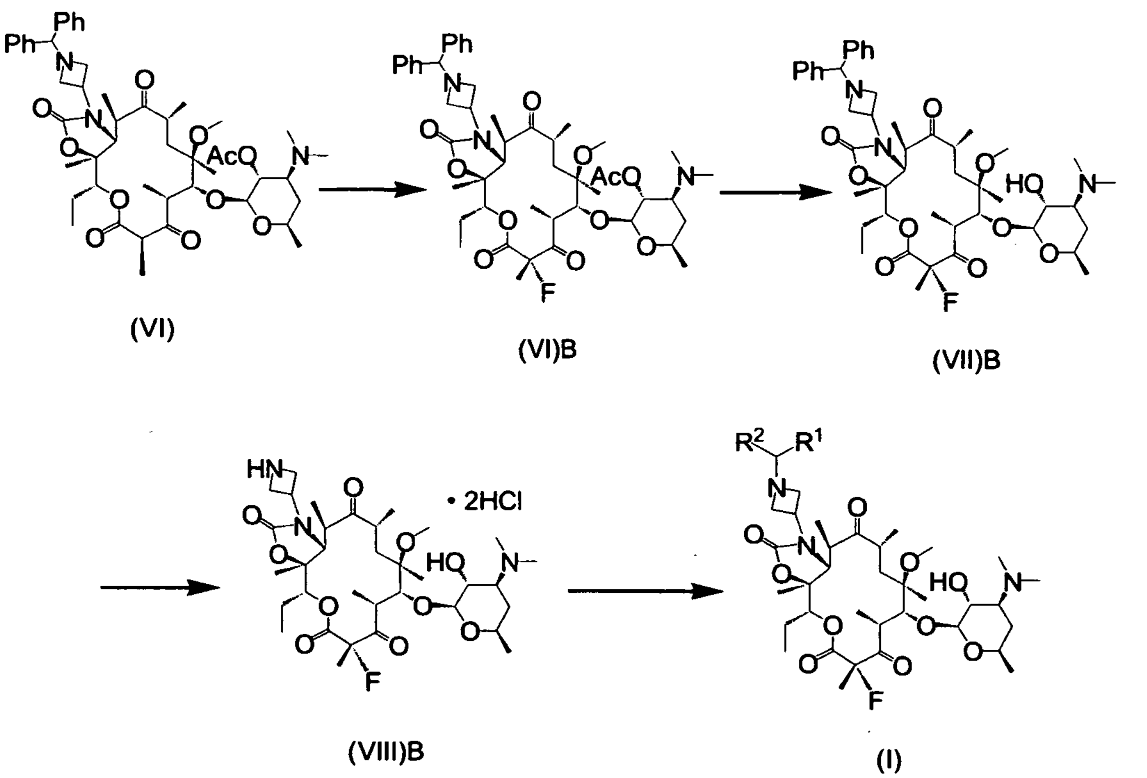

(VI) (VI)B (VII)B

(VIII)B (I)

Scheme 2 depicts how to make a compound of Formula (I) that is fluorinated at the C-2 position. This is done by fluorinating (VI) at the C-2 position to make (VI)B by first generating an anion/enolate at the C-2 carbon by reaction of (VI) with a base such as sodium hydride, potassium diisopropylamide, or lithium hexamethyldisilazide in a solvent such as DMF at about -50 to -30°C for about 10 to 30 minutes. This anion can then be reacted with a variety of electrophiles including an electropositive fluorinating agent such as Selectfluor®. After making (VI)B, the same procedures described

for Scheme 1 may be used to arrive at a compound of Formula (I).

The following compounds are useful intermediates in preparing compounds of Formula (I).

Preparation A

**1-Benzhydryl-azetidin-3-ylamine:**

Methanesulfonic acid 1-benzhydryl-azetidin-3-yl ester (14.8 g, 47 mmol, Oakwood Products) was dissolved in anhydrous DMF (60 mL), and to this solution was added sodium azide (9.0 g, 138 mmol). The mixture was heated (80˚C) for 18 hours, cooled to room temperature, then treated with water (20 mL) and saturated aqueous $NaHCO_3$ (20 mL). The resulting mixture was extracted with DCM (4 x 60 mL) and the organic layer dried over $Na_2SO_4$. Solvent removal yielded a crude oil (11 g) that was redissolved in THF (85 mL) and treated with triphenylphosphine (15 g, 57 mmol). After stirring at room temperature (30 minutes; gas evolution and some exotherm noted), the mixture was heated to reflux (6 hours), then cooled back down to room temperature before adding $NH_4OH$ (7 mL) and refluxing again (5 hours). After cooling to room temperature, the solvent was removed and the residue treated with 3N HCl (35 mL) giving a pH 1. The resulting acidic solution was extracted with DCM (3 x 50 mL) and the organic layers were discarded. The aqueous layer was then basified with solid potassium hydroxide to pH 10 and then extracted again with DCM (3 x 75 mL). The aqueous layer was then saturated with solid sodium chloride and re-extracted with DCM (3 x 75 mL). The combined organic layers were dried over $Na_2SO_4$, filtered, and concentrated to yield crude 1-benzhydryl-azetidin-3-ylamine (9.0 g, 37.8 mmol). MS (ESI+) for *m/z* $(M+H)^+$ 239.

Preparation B

**3-Descladinosyl-11,12-dideoxy-6-O-methyl-3-oxo-12,11-(oxycarbonyl-(azetidin-3-yl)imino)-erythromycin A Dihydrochloride (VIII)**

(Steps 1-5 of Scheme 1):

Step 1: DBU (10 mL, 67 mmol) was added to 1-benzhydryl-azetidine-3-ylamine (9.0 g, 37.8 mmol) dissolved in anhydrous MeCN (100 mL) at room temperature. To this solution was added 11-deoxy-2',4"-diacetyl-10,11-didehydro-12-O-((1H-imidazol-1-yl)-carbonyl)-6-O-methyl-erythromycin A (III) (32.5 g, 35.8 mmol) and the resulting mixture was heated (50˚C) for 7.5 hours, then allowed to stir overnight at room temperature. A white precipitate was collected by filtration (21 g, 19.5 mmol). The filtrate was concentrated to about 25 mL before adding water (20 mL), saturated aqueous $NaHCO_3$ solution (30 mL), and extracting the resulting mixture with DCM (50 mL). The organic layer was washed with saturated aqueous $NaHCO_3$ solution, dried over $Na_2SO_4$, and concentrated under vacuum to a tan foam (IV). MS (ESI+) for *m/z* 540 $(M/2+H)^+$.

Step 2: The product from Step 1 was dissolved in EtOH (100 mL) and 2N HCl (100 mL), heated (40˚C) for 3 hours, then cooled to 30˚C and stirred overnight. The resulting mixture was concentrated under vacuum, maintaining the bath at 28˚C, to about half of the original volume. To the resulting concentrate was added DCM (100 mL), followed by careful addition of solid potassium carbonate to basify the aqueous layer (to pH 10). The organic layer was separated, and the aqueous layer was re-extracted with DCM (3 x 100 mL). The combined organic layers were dried over $Na_2SO_4$ and concentrated to a white foam (V). MS (ESI+) for *m/z* 440 $(M/2+H)^+$.

Step 3a: The crude product from Step 2 was re-dissolved in anhydrous DCM (200 mL). To this was added anhydrous DMSO (20 mL, 282 mmol), pyridinium trifluoroacetate (15 g, 77.7 mmol), and finally EDC (30 g, 156.5 mmol). The mixture was stirred at room temperature for 3 hours and then treated with saturated aqueous $NaHCO_3$ solution (30 mL) and water (30 mL). The reaction mixture was placed in a separatory funnel and the layers allowed to separate for several hours. The aqueous layer was re-extracted with DCM (3 x 75 mL) and the combined organic layers were washed with water (50 mL). After drying the organics over $Na_2SO_4$, the solvent was removed under vacuum to yield a yellow foam (VI). MS (ESI+) for *m/z* 439 $(M+H)^+$.

Step 4: The yellow foam from Step 3a was re-dissolved in MeOH (200 mL) and heated (50˚C) for 24 hours, then concentrated under vacuum to dryness. This solid material was rinsed with hexanes/diethyl ether (2/1), treated with MeOH (50 mL) and the resulting slurry heated on a steam bath up to the boiling point, then cooled to room temperature. The resulting white solid (VII) was filtered and dried under vacuum (10.5 g, 12.6 mmol, 35% yield over four steps). MS (ESI+) for m/z 418 $(M/2+H)^+$. $^1$H NMR ($CDCl_3$) $\delta$ 7.41 (m, 4 H), 7.21 (m, 4 H), 7.16 (m, 2 H), 4.88 (br d, 1 H), 4.60 (s, 1 H).

The following Step 3b is an alternative route which may be used in place of Steps 3a and 4.

Step 3b: NCS (9.94 g, 74.5 mmol) was dissolved in DCM (175 mL), and the resulting solution was cooled to -10˚C. Dimethyl sulfide (5.09 g, 81.9 mmol) was added while keeping the temperature under -10˚C. The resulting mixture was stirred for 1 hour at -10˚C. Intermediate (V) (35 g, 37.2 mmol) (wherein Bz was used in place of Ac) in dichloromethane

(175 mL) at -10˚C was slowly introduced while keeping the reaction temperature under -10˚C. The resulting mixture was stirred for 1 hour at -10˚C. Triethylamine (5.92 mL, 42.4 mL) was added slowly, again keeping the temperature below -10˚C. After stirring for 1 hour below -10˚C, the reaction was quenched into 350 mL of saturated aqueous $NaHCO_3$ solution and DCM (350 mL). The organic layer was split, washed with saturated aqueous $NaHCO_3$ solution (350 mL), and concentrated to a low stirrable volume (approximately 100 mL). The residual DCM was displaced with methanol (final methanol volume 350 mL). The resulting mixture was heated in methanol at reflux for 12 hours, and concentrated to approximately 100 mL. The mixture was cooled to 5˚C, and filtered. The product (VII) was collected by filtration to give 20.5 g (24.6 mmol, 66% yield).

Step 5: The white solid from Step 4 was dissolved in MeOH and treated with concentrated aqueous HCl (2.4 mL, 28 mmol). To this was added Pearlman's catalyst (palladium hydroxide on carbon, 20% weight Pd, approximately 6 g). The scurry was filtered. To the filtrate was added Pearlman's catalyst (palladium hydroxide on carbon, 10% weight Pd, approximately 3 g ), and the slurry was subjected to hydrogen gas (40 to 50 psi) in a pressure reactor while heating (35˚ - 45˚C) for up to 2 hours. The pressure reactor was cooled to room temperature, purged with nitrogen, and the catalyst was filtered off. The filtrate was concentrated under vacuum to a smaller volume (approximately 50 mL), and THF (approximately 200 mL) was added. The solution was concentrated until the residual water and methanol were removed (confirmed with GC-headspace and Karl Fisher test, respectively), and further concentrated to 60 mL as the final volume. The resulting slurry was granulated at 10˚C for a minimum of 2 hour and filter. The solid collected was dried under full vacuum to yield the title compound (14 g) (VIII). MS (ESI+) for m/z 335 $(M/2+H)^+$. $^1H$ NMR ($CD_3OD$) δ 4.71-4.59 (m, 2 H), 4.48 (br t, 1 H), 4.33 (d, 1 H), 4.27 (d, 1 H), 4.16 (br t, 1 H).

Preparation C

**3-Hydroxy-[1,5]-naphthyridin-4-carbaldehyde:**

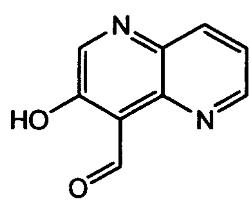

Step 1 (Enamine Formation): [(E)-2-(3-Methoxy-[1,5]-naphthyridin-4-yl-vinyl]-dimethyl-amine.

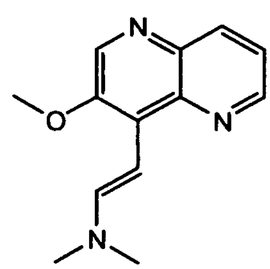

To a 250 mL round-bottomed flask fitted with an overhead stirrer, reflux condenser, internal temperature probe, heating mantle and nitrogen inlet/outlet were charged 3-methoxy-4-methyl-[1,5]-naphthyridine (10.01g, 57.46 mmol), DMF (100 mL, 10 vol), DMF/DMA (13.73 g, 115.22 mmol) and potassium tert-butoxide (3.23 g, 28.78 mmol) and heated with stirring to 120˚C for 20 hours. The reaction mixture was sampled by GCMS and found to contain 4% starting material and 96% product. The mixture was then cooled to 65˚C and distilled under full vacuum to approximately 50 mL. This mixture was carried forward to the Step 2 oxidation. An analytical sample was obtained by filtration of the mixture. $^1H$-NMR ($CDCl_3$, 400 MHz) δ 8.824 (1H, dd, J = 4.14, 2.07 Hz), 8.604 (1H, s), 8.315 (1H, d, J = 13.69 Hz), 8.207 (1H, dd, J = 8.71, 2.07 Hz), 7.434 (1H, dd, J = 8.29, 3.73 Hz), 6.209 (1H, d, J = 13.69 Hz), 4.083 (3H, s), 3.022 (6H, s).

Step 2 (Sodium Periodate Oxidation): 3-Methoxy-[1,5]-naphthyridin-4-carbaldehyde.

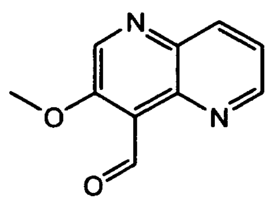

To a 250 mL round-bottomed flask fitted with an overhead stirrer, reflux condenser, internal temperature probe and nitrogen inlet/outlet was charged a slurry of sodium periodate (24.7 g, 115.48 mmol) in water (150 mL). The product mixture from the enamine formation in step 1 (~50 mL solution, theoretical 13.16 g, 57.40 mmol of the enamine) was charged to the periodate slurry resulting in an exotherm from 20˚C to 50˚C. The solution initially turned purple and then red/brown. The reaction mixture was cooled to 25˚C and stirred for 2 hours and then sampled for completion by GCMS. Analysis indicated that no starting material remained and the product accounted for 82% of the mixture. The solids were filtered off and the cake washed with EtOAc (200 mL). The phases were split and the aqueous/DMF layer was extracted with EtOAc (3 x 200 mL). The combined organic layers were then dried over $MgSO_4$, filtered and concentrated under vacuum (40˚C, 60 mbar) to a brown solid. The crude product was then taken back up in EtOAc (30 mL) and recrystallized at -10˚C. The solid was filtered off, washed with cold EtOAc (5 mL) and dried to afford the desired product (5.34 g, 28.4 mmol, 49%). $^1$H-NMR ($CDCl_3$, 300 MHz). δ 11.352 (1H, s), 9.094 (1H, s), 9.051 (1H, dd, J = 4.36, 1.86 Hz), 8.425 (1H, dd, J = 8.41, 1.55 Hz), 7.613 (1H, dd, J = 8.41, 4.05 Hz), 4.246 (3H, s).

Step 3 (De-Methylation): 3-Hydroxy-[1,5]-naphthyridin-4-carbaldehyde.

The product from Step 2 (50 g, 0.266 mol) was combined in a flask with LiCl (11.3 g), DMF (200 mL) and heated at reflux for 20 hours. The reaction mixture was distilled under vacuum to about half of the original DMF volume. Water (250 mL) was then added (the lithium salt, the color and impurities were dissolved in water) followed by MeOH (1000 mL) and the product precipitated to provide 27.7 g (60% yield) of the title compound. $^1$H-NMR ($D_2O$, 400 MHz) δ 10.045 (1H, s), 8.54-8.46 (1H, m), 8.46-8.40 (1H, m), 8.319 (1H, s), 7.56-7.46 (1H, m).

Preparation D

**4-Formyl-1,5-naphthyridin-3-yl Pivalate**

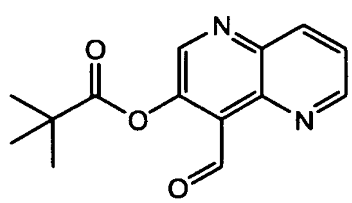

In a flask was added hydroxy aldehyde naphthyridine (10 g, 1.0 equiv ), THF (100 mL), triethylamine (1 equivalent), and pivaloyl chloride (1 equivalent) at room temperature. The reaction was stirred for 1 hour. The reaction mixture was filtered through celite and rinsed with THF (20 mL). The filtrate and rinse were combined and stripped to a low stirrable volume. Heptanes (200 mL) was added and the mixture was heated to reflux. The reaction mixture was cooled to 85˚C and filtered hot. The flask was rinsed and filtered with hot heptanes (100 mL). The filtrate and rinse were combined and stripped to approximately 100 mL. The mixture was cooled in an ice bath and the product crystallized out. The product was filtered and rinsed with heptane (20 mL), dried under vacuum and isolated to provide 9.6 g (65%) of the title compound. $^1$H-NMR ($CDCl_3$, 400 MHz) δ 11.32 (1H, s), 9.08 (1H, dd, J = 4.15, 1.66 Hz), 8.85 (1H, s), 8.50 (1H, dd, J = 8.29, 1.66 Hz), 7.73 (1H, dd, J = 8.71, 4.15 Hz), 1.47 (9H, s). $^{13}$C-NMR ($CDCl_3$) δ 27.32, 39.60, 124.48, 127.73, 137.77, 142.39, 142.47, 143.64, 149.00, 152.41, 176.39, 191.20.

## Examples

The present invention will be further illustrated by means of the following non-limiting examples.

Example 1

**3-Descladinosyl-11,12-dideoxy-6-O-methyl-3-oxo-12,11-(oxycarbonyl-(1-((3-hydroxy-[1,5]-naphthyridin-4-yl) methyl)-azetidin-3-yl)-imino)-erythromycin A Fumarate**

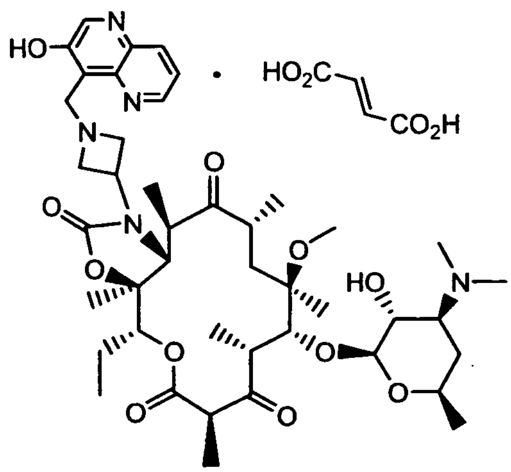

3-Hydroxy-[1,5]-naphthyridin-4-carbaldehyde (10 g, 57.6 mmol) and 3-descladinosyl-11,12-dideoxy-6-O-methyl-3-oxo-12,11-(oxycarbonyl-(azetidin-3-yl)-imino)-erythromycin A dihydro-chloride (41 g, 55.3 mmol) were combined in THF (410 mL), then TEA (21.9 mL, 157 mmol) was added. The mixture was stirred for 30 minutes, followed by the addition of pivalic acid (21.4 g, 210 mmol). The mixture was heated to reflux and approximately 600 mL of solvent was removed by concentration under atmospheric pressure to ensure complete removal of water (an additional 400 mL of anhydrous THF was added during the concentration). The mixture was then cooled to 20 - 25°C, and transferred to a reaction flask that contained sodium triacetoxyborohydride (55.5 g, 262 mmol), acetonitrile (164 mL) and ethyl acetate (1230 mL) at 20 - 25°C under agitation. After stirring for 30 minutes, 5% aqueous sodium bicarbonate (205 mL) was added. The layers were separated. The aqueous layer was extracted with ethyl acetate (205 mL). The combined organic phases were treated with anhydrous magnesium sulfate (41 g), then active carbon (10.3 g) was added. The mixture was filtered, and fumaric acid (4.26 g, 36.7 mmol) was added. The resulting mixture was stirred for a minimum of 48 hours. The product was collected by filtration and dried under vacuum at 35°C to yield 28.72 g (55.1 %) of the title compound. LCMS: Positive ion scan: 414 (M/2 + 1), 827 (M + 1). $^1$H NMR (400 MHz, $CD_3OD$): [selected peaks only] □ 8.88 (m, 1H); 8.67 (s, 1H); 8.30 (dd, *J* = 2, 9 Hz, 1H); 7.54 (m, 1H); 6.66 (s, 2H, fumarate olefin); 2.79 (s, 6H); 2.59 (s, 3H); 0.98 (d, *J* = 7 Hz, 3H); 0.85 (t, *J* = 7 Hz, 3H).

Example 2

**3-Descladinosyl-11,12-dideoxy-6-O-methyl-3-oxo-12,11-(oxycarbonyl-(1-((3-hydroxy-[1,5]-naphthyridin-4-yl)-methyl)-azetidin-3-yl)-imino)-erythromycin A** (free base)

3-Hydroxy-[1,5]-naphthyridin-4-carbaldehyde (11 g, 63 mmol) was dissolved in anhydrous acetonitrile (1400 mL) in a 3-L 3-neck round bottom flask, followed by addition of triethylamine (44 mL, 316 mmol) and powdered molecular sieves (47 g, 4 Angstrom). The mixture was mechanically stirred at 50°C for 30 min, followed by addition of 3-descladinosyl-11,12-dideoxy-6-O-methyl-3-oxo-12,11-(oxycarbonyl-(azetidin-3-yl)-imino)-erythromycin A dihydrochloride (46.8 g, 63.2 mmol). The resulting mixture was stirred for another 1 hr at 50°C, after which time it was cooled to room temperature, and treated carefully with sodium triacetoxyborohydride (67 g, 316 mmol), added in equal portions over a 40 min period. The resulting mixture was stirred at room temperature overnight. Celite (50 g) and MeOH (800 mL) were subsequently added to the reaction mixture, and the solids removed by filtration through a coarse-fritted funnel pre-packed with Celite, rinsed several times with MeOH (approximately 4 L). The organic filtrate was concentrated on a

rotary evaporator to a smaller volume (approximately 200 mL), diluted with dichloromethane (400 mL), transferred to a beaker, treated with water (300 mL) and cautiously treated with saturated aqueous sodium bicarbonate solution (300 mL). The resulting solution was subsequently transferred to a separatory funnel and extracted with dichloromethane (5 x 600 mL, being careful to avoid pressure build up). The organic extracts were combined, dried over anhydrous sodium sulfate, filtered, and concentrated to dryness in vacuo to give crude product (56 g) as a brown solid. This solid was then dissolved in dichloromethane and chromatographed on silica gel, eluting isocratically with ethyl acetate/MeOH/concentrated aqueous ammonium hydroxide (89/10/1) to remove some of the colored impurities and achieve an enriched fraction. The enriched fraction was dried in vacuo, re-dissolved in dichloromethane, and then purified again on silica gel using a gradient elution of solvent A (ethyl acetate) and solvent mixture B (ethyl acetate/MeOH/concentrated aqueous ammonium hydroxide in a respective ratio of 89/10/1), starting with 100% solvent A and ending with a 95/5 ratio of solvent A to solvent mixture B. This resulted in approximately 23 g of purified title compound as a free base. LCMS: Positive ion scan: 414 (M/2 + 1), Negative ion scan: 825 (M - 1), $^1$H NMR (400 MHz, $CDCl_3$): [selected peaks only] □ 8.79 (m, 1H); 8.59 (s, 1H); 8.22 (dd, *J* = 2, 8 Hz, 1H); 7.37 (m, 1H); 4.87 (br d, 1H); 4.70 (m, 2H); 4.25 (t, *J* = 8 Hz, 2H); 2.65 (s, 3H); 2.23 (s, 6H); 0.98 (d, *J* = 7 Hz, 3H); 0.82 (t, *J* = 7 Hz, 3H).

The following examples 3 to 10 may be similarly prepared by one skilled in the art using the methods described herein.

Example 3

**3-Descladinosyl-11,12-dideoxy-6-O-methyl-3-oxo-12,11-(oxycarbonyl-(1-((3-hydroxy-[1,6-]naphthyridin-4-yl)-methyl)-azetidin-3-yl)-imino)-erythromycin A Fumarate**

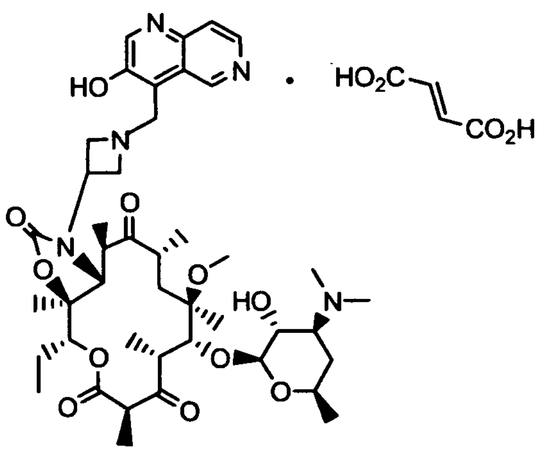

Same as procedure described in Example 1, but using 3-hydroxy-[1,6]-naphthyridin-4-carbaldehyde instead of 3-hydroxy-[1,5]-naphthyridin-4-carbaldehyde, which may be made analogously.

Example 4

**3-Descladinosyl-11,12-dideoxy-6-O-methyl-3-oxo-12,11-(oxycarbonyl-(1-((3-hydroxy-[1,7]-naphthyridin-4-yl)-methyl)-azetidin-3-yl)-imino)-erythromycin A Fumarate**

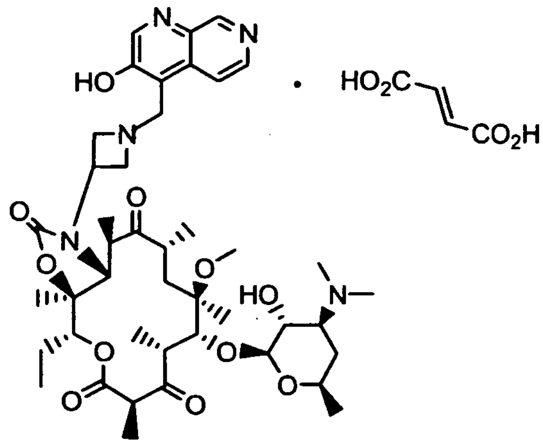

Same as procedure described in Example 1, but using 3-hydroxy-[1,7]-naphthyridin-4-carbaldehyde instead of 3-hydroxy-[1,5]-naphthyridin-4-carbaldehyde, which may be made analogously.

Example 5

**3-Descladinosyl-11,12-dideoxy-6-O-methyl-3-oxo-12,11-(oxycarbonyl-(1-((3-hydroxy-8-methyl-[1,5]-naphthyridin-4-yl)-methyl)-azetidin-3-yl)-imino)-erythromycin A Fumarate**

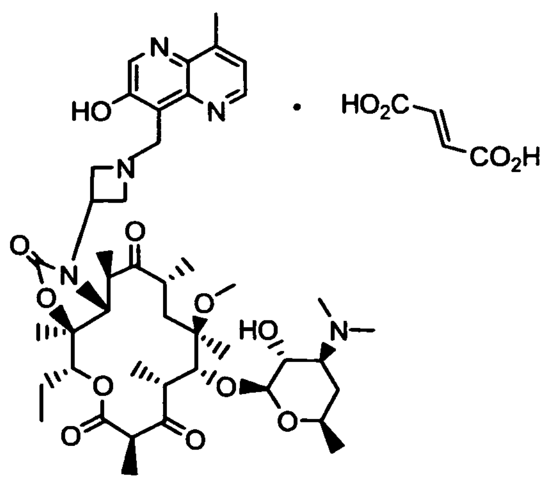

Same as procedure described in Example 1, but using 3-hydroxy-8-methyl-[1,5]-naphthyridin-4-carbaldehyde instead of 3-hydroxy-[1,5]-naphthyridin-4-carbaldehyde, which may be made analogously.

Example 6

**3-Descladinosyl-11,12-dideoxy-6-O-methyl-3-oxo-12,11-(oxycarbonyl-(1-((3-hydroxy-6-methyl-[1,5]-naphthyridin-4-yl)-methyl)-azetidin-3-yl)-imino)-erythromycin A Fumarate**

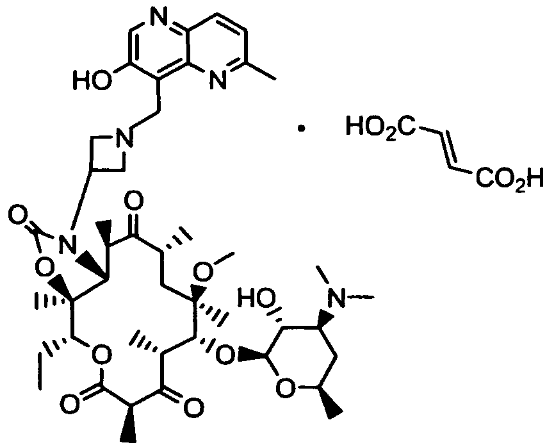

Same as procedure described in Example 1, but using 3-hydroxy-6-methyl-[1,5]-naphthyridin-4-carbaldehyde instead of 3-hydroxy-[1,5]-naphthyridin-4-carbaldehyde, which may be made analogously.

Example 7

**3-Descladinosyl-11,12-dideoxy-6-O-methyl-3-oxo-12,11-(oxycarbonyl-(1-((3-hydroxy-7-methyl-[1,5]-naphthyridin-4-yl)-methyl)-azetidin-3-yl)-imino)-erythromycin A Fumarate**

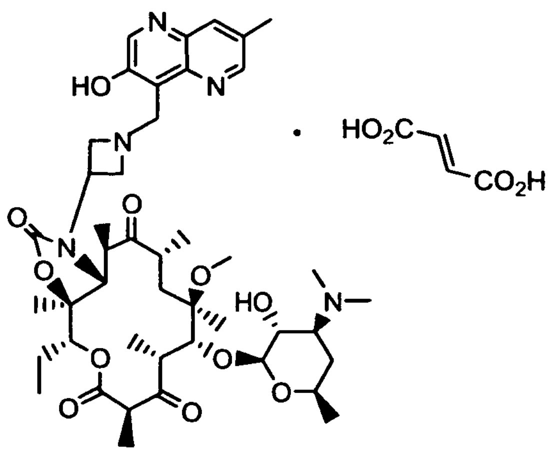

Same as procedure described in Example 1, but using 3-hydroxy-7-methyl-[1,5]-naphthyridin-4-carbaldehyde instead of 3-hydroxy-[1,5]-naphthyridin-4-carbaldehyde, which may be made analogously.

Example 8

**3-Descladinosyl-11,12-dideoxy-6-O-methyl-3-oxo-12,11-(oxycarbonyl-(1-((3-hydroxy-2-methyl-[1,5]-naphthyridin-4-yl)-methyl)-azetidin-3-yl)-imino)-erythromycin A Fumarate**

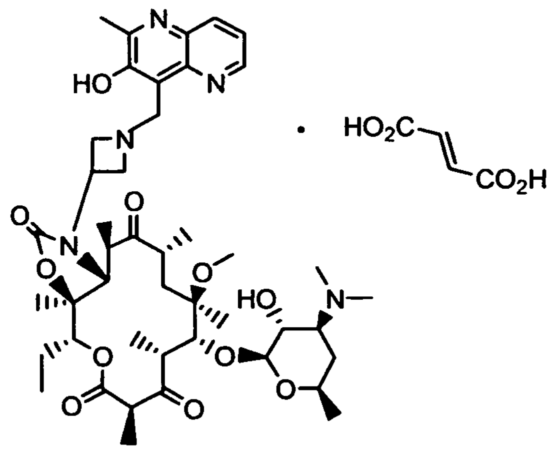

Same as procedure described in Example 1, but using 3-hydroxy-2-methyl-[1,5]-naphthyridin-4-carbaldehyde instead of 3-hydroxy-[1,5]-naphthyridin-4-carbaldehyde, which may be made analogously.

Example 9

**2-Fluoro-3-descladinosyl-11,12-dideoxy-6-O-methyl-3-oxo-12,11-(oxycarbonyl-(1-((3-hydroxy-[1,5]-naphthyridin-4-yl)-methyl)-azetidin-3-yl)-imino)-erythromycin A Fumarate**

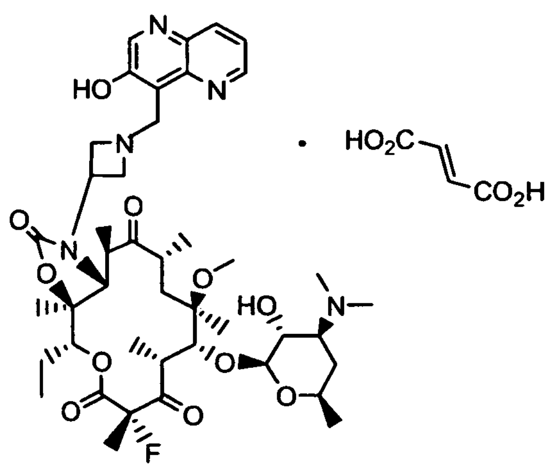

Same as procedure described to make Example 1, but first fluorinating C-2 of intermediate (VI) according to Scheme 2 and coupling (VIII)B to 3-Hydroxy-[1,5]-naphthyridin-4-carbaldehyde.

Example 10

**3-Descladinosyl-11,12-dideoxy-6-O-methyl-3-oxo-12,11-(oxycarbonyl-(1-(1-(3-hydroxy-[1,5]-naphthyridin-4-yl)-ethyl)-azetidin-3-yl)-imino)-erythromycin A Fumarate**

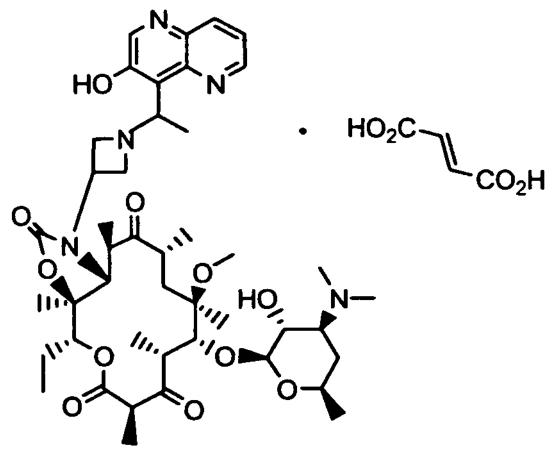

Analogous to Preparation 28 for Example 1.25 described in WO 2006/067589, published June 29, 2006, but using 1-(3-hydroxy)-[1,5]-naphthydrin-4-yl-ethanone instead of 9-[1,8]-naphthydrin-4-yl-ethanone.

Biologic Properties

The compounds of Formula (I) are useful for the prevention and/or treatment of bacterial infections alone or in combination with other antibacterial agents.

In some embodiments, compounds of the invention exhibit a broad spectrum of antibacterial activity and/or are effective against a variety of infectious strains of interest. For example, using a standard microtiter broth serial dilution test, compounds of the invention have been found to exhibit useful levels of activity against a wide range of pathogenic microorganisms, including strains of *Staphylococcus aureus, S. pneumoniae, M. catarrhalis, S. pyogenes,* and *H. influenzae,* including resistant strains. Compounds of the invention can therefore be used, *e.g.*, for treating and/or preventing a variety of diseases caused by pathogenic bacteria in human beings and animals.

Bacterial strains that contain the gene with the designation of ermA, ermB, or ermC are resistant to some macrolides, lincosamides, and streptogramin B antibiotics due to modifications (methylation) of 23S rRNA molecules by an Erm methylase, thereby generally attenuating binding by members of all three antibiotic classes. Other strains containing macrolide efflux genes have also been described. For example, msrA encodes a component of an efflux system in staphylococci that prevents accumulation of some macrolides and streptogramins, while mefA/E encodes a transmembrane protein that appears to efflux only macrolides. Inactivation of macrolide antibiotics can occur and can be mediated by either a phosphorylation of the 2'-hydroxyl (mph) or by cleavage of the macrocyclic lactone (esterase). "AcrAB" or "AcrAB-like" indicates that an intrinsic multidrug efflux pump exists in the strain.

Activity against bacterial and protozoal pathogens can be demonstrated by a compound's ability to inhibit growth of defined strains of pathogens. The assay described herein includes a panel of bacterial strains assembled to include a variety of target pathogenic species, including representatives of macrolide resistance mechanisms that have been characterized. Bacterial pathogens that comprise the screening panel are shown in the tables below. In many cases, both the macrolide-susceptible parent strain and the macrolide-resistant strain derived from it are available to provide a more accurate assessment of the compound's ability to circumvent the resistance mechanism. The assay is performed in microtiter trays and interpreted according to Methods for Dilution Antimicrobial Susceptibility Tests for Bacteria that Grow Aerobically; Approved Standard-Seventh Edition (M7-A7) and Performance Standards for Antimicrobial Susceptibility Testing; 17th Informational Supplement (M100-S17), published by The Clinical and Laboratory Standards Institute (CLSI) guidelines; the MIC is used to compare strains. Compounds are initially dissolved in DMSO as 40 mg/ml stock solutions.

| Strain Designation | Macrolide Resistance Mechanism(s) |
|---|---|
| *Streptococcus pyogenes* 1079 | ermB |
| *Streptococcus pneumoniae* 1016 | (susceptible) |
| *Streptococcus pneumoniae* 1095 | ermB |
| *Streptococcus pneumoniae* 1175 | mefA |
| *Haemophilus influenzae* 1218 | (susceptible) |

Table 1 lists in vitro data demonstrating the antibacterial activity of Example 1 and comparative drugs erythromycin and telithromycin against key respiratory pathogens. Macrolides are used for short-term therapy (<10 days) for mild-to-moderate community-acquired pneumonia, and ≤ 5 days for acute exacerbations of chronic bronchitis, sinusitis, acute otitis media, and tonsillitis/pharyngitis. Key pathogens in this group include *S. pneumoniae, S. pyogenes, H. influenzae,* and *M. catarrhalis.* MICs (Range, $MIC_{50}$, $MIC_{90}$) for Example 1 and comparator agents against these key pathogens is shown below. Example 1 has in vitro activity against macrolide-resistant strains of *S. pneumoniae* (ermB, mefA and ermB/mefA) that is comparable to telithromycin. The activity of Example 1 against *S. pyogenes* strains containing mefA is similar to that of telithromycin. When $MIC_{90s}$ against ermB-containing *S. pyogenes* isolates are compared, however, Example 1 demonstrated a 16-fold improvement over telithromycin. The $MIC_{90s}$ of Example 1 and telithromycin against 82 telithromycin-sensitive *H. influenzae* strains were both 4 μg/mL. When evaluated against 12 telithromycin-intermediate *H. influenzae* isolates, the $MIC_{90s}$ of Example 1 and telithromycin were both 8 μg/mL. Against *M. catarrhalis,* the in vitro activity of Example 1 is slightly better than that of telithromycin ($MIC_{90}$ of 0.06 vs. 0.25 μg/mL, respectively).

Table 1. In vitro activity of Example 1 and comparator agents against selected human respiratory pathogens.

| Organism (n) | Genotype | Compound | MIC (μg/mL) | | |
|---|---|---|---|---|---|
| | | | Range | $MIC_{50}$ | $MIC_{90}$ |
| *S. pneumoniae* (34) | *ermB* | Example 1 | ≤ 0.0625 - 2 | ≤ 0.0625 | 1 |
| | | erythromycin | 8 - >64 | >64 | >64 |
| | | telithromycin | ≤ 0.0625 - 1 | ≤ 0.0625 | 0.5 |
| | | | | | |
| *S. pneumoniae* (39) | *mefA* | Example 1 | ≤ 0.06 - 1 | 0.125 | 0.5 |
| | | erythromycin | 2 - 16 | 8 | 16 |
| | | telithromycin | ≤ 0. 06 - 0. 5 | 0.125 | 0.5 |
| | | | | | |
| *S. pneumoniae* (45) | *ermBl* *mefA* | Example 1 | ≤ 0.0625 - 1 | 0.5 | 1 |
| | | erythromycin | 16 - >64 | >64 | >64 |
| | | telithromycin | ≤ 0.0625 - 1 | 0.5 | 0.5 |
| | | | | | |
| *S. pyogenes (25)* | *ermB* | Example 1 | ≤ 0.06 - 4 | 1 | 2 |
| | | amoxicillin | 0.002 - 0.0156 | 0.0078 | 0.0078 |
| | | erythromycin | 4 - >64 | >64 | >64 |
| | | telithromycin | ≤ 0.06 - 64 | 8 | 32 |
| | | | | | |
| *S. pyogenes (23)* | *mefA* | Example 1 | 0.5 - 1 | 0.5 | 1 |
| | | amoxicillin | 0.004 - 0.03 | 0.008 | 0.016 |
| | | erythromycin | 8 - 32 | 8 | 16 |
| | | telithromycin | 0.25 - 2 | 0.5 | 0.5 |
| | | | | | |
| *H. influenzae* (82) | Telithromycin (susceptible) | Examples 1 | 1 - 8 | 2 | 4 |
| | | erythromycin | 1-16 | 8 | 16 |
| | | telithromycin | 0.25 - 4 | 1 | 4 |
| | | | | | |

(continued)

| Organism (n) | Genotype | Compound | MIC ($\mu$g/mL) | | |
|---|---|---|---|---|---|
| | | | Range | $MIC_{50}$ | $MIC_{90}$ |
| *H. influenzae* (12) | Telithromycin (intermediate) | Example 1 | 4 - 8 | 4 | 8 |
| | | erythromycin | 8 - 16 | 16 | 16 |
| | | telithromycin | 8 | 8 | 8 |
| | | | | | |
| *M. catarrhalis* (30) | | Example 1 | 0.008 - 0.25 | 0.03 | 0.06 |
| | | erythromycin | 0.03 - 1 | 0.25 | 0.25 |
| | | telithromycin | 0.006 - 0.5 | 0.125 | 0.25 |

In vitro activity of Example 1 against atypical pneumonia pathogens

Macrolide class antibiotics have been the therapy of choice for pneumonias caused by organisms classified as atypical pathogens, including *M. pneumoniae, L. pneumophila, Mycobacterium avium,* and *C. pneumoniae.* Table 2 shows the in vitro activity profile of Example 1 against one of these species (*L. pneumophila*). Example 1 demonstrated equivalent activity to azithromycin and telithromycin against ten isolates of *L. pneumophila*.

Table 2. In vitro antibacterial activity of Example 1 against an atypical pneumonia-causing pathogen.

| Organism (n) | Genotype | Compound | MIC ($\mu$g/mL) | | |
|---|---|---|---|---|---|
| | | | Range | MIC50 | MIC90 |
| *L. pneumophila* (10) | NA | Example 1 | 0.008 - 0.03 | 0.016 | 0.03 |
| | | azithromycin | 0.03 - 0.06 | 0.06 | 0.06 |
| | | telithromycin | 0.016 - 0.03 | 0.016 | 0.016 |

In some embodiments, compounds of the invention can be used against macrolide-resistant or multi-drug resistant bacterial strains. For example, in some embodiments, compounds of the invention exhibit favorable activity against resistant *S. pyogenes* strains, including methylation-based resistance such as ermB. Thus, the invention includes using the compounds to take advantage of such favorable properties.

In vitro assessment of human liver metabolism and CYP3A Inhibition

The compounds of Formula (I) of the present invention have highly desirable metabolic properties as determined by in vitro assessment of human liver metabolism and CYP3A Inhibition.

The 3-hydroxy substituted analog (Example 1) demonstrated a lower human liver microsomal (HLM) extraction ratio (ER) compared to the unsubstituted (Comparative Example A) or 3-bromine-substituted (Comparative Example B) analogs, disclosed as Examples 1.26 and 1.106, respectively, in WO 2006/067589, published June 29, 2006. Human liver microsomal extraction ratio is an indication of how quickly the compound is metabolized by drug metabolizing enzymes, especially cytochrome P450s in the human liver. Example 1 has a lower extraction ratio relative to Comparative Examples A and B, and hence would be expected to exhibit a lower clearance in humans and thus result in lower dose required for the same efficacy. The results are illustrated in Table 3 below in the column labeled "HLM ER".

Example 1 also demonstrated a decreased affinity for cytochrome P450 3A (CYP3A) compared to the unsubstituted (Comparative Example A) or bromine-substituted (Comparative Example B) analogs, as evidenced by its higher $K_I$ value shown in Table 3 below. CYP3A is the major drug-metabolizing enzyme in human liver, catalyzing metabolism of an estimated 50% of all drugs. Inhibition of CYP3A can lead to drug-drug interactions. Most commercially available macrolides/ketolides are time-dependent inhibitors of CYP3A. Time-dependent inhibition (TDI) was assessed in vitro using human liver microsomes and a probe substrate for CYP3A activity (midazolam). Test compounds with lower $K_I$ values and/or higher $k_{inact}$ values have greater affinity for CYP3A and thereby greater potential for drug-drug interactions. As shown in Table 3, hydroxylation at the 3-position of the [1,5]-naphthyridine (Example 1) resulted in an approximately 10-fold increase in $K_I$ in the CYP3A TDI assay compared to the unsubstituted (Comparative Example A) or bromine-substituted (Comparative Example B) analogs. The increased $K_I$ observed for Example 1 results in a lower predicted fold change in area under the curve (AUC) for a coadministered CYP3A substrate compared to Comparative Examples A and B.

Table 3

| 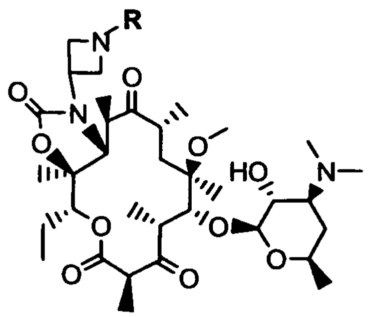 | | | | CYP3A Time-Dependent Inhibition | |
|---|---|---|---|---|---|
| **Compound Name** | **R** | **HLM** | **ER $K_I$ (μM)** | **$k_{inact}$ ($min^{-1}$)** | **Predicted fold-change in AUC of CYP3A substrate** |
| Example 1 | 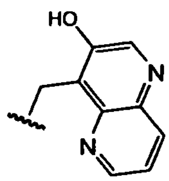 | <0.32 | 24 | 0.024 | 1.3 |
| Comparative Example A | 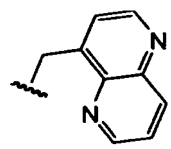 | 0.70 | 2.0 | 0.028 | 5.4 |
| Comparative Example B | 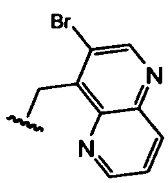 | 0.91 | 2.8 | 0.029 | 4.2 |

The following assay procedures were used to determine the HLM ER (human liver microsomes extraction ratio) and CYP3A time-dependent inhibition for a representative example of a compound of Formula (I) of the invention and two comparative examples.

Assay Procedures for in vitro assessment of human liver metabolism and CYP3A Inhibition

HLM ER: (human liver microsomes extraction ratio). This assay measures clearance of compound over time in the human liver microsome system due to NADPH-dependent oxidative metabolism. These data are used to estimate the human hepatic metabolic clearance for a given compound. The results can range from 0.32 to 0.99. A value of <0.32 represents the lower limit of the assay and would indicate a low clearance compound. Values in the range 0.33 to 0.70 indicate moderate clearance, and values >0.70 indicate high clearance.

Assay Procedure: Macrolide test compounds were incubated at a final concentration of 1 μM in human liver microsomes (final CYP concentration = 0.50 μM) in 0.1 M potassium phosphate buffer (pH = 7.4) at 37°C with 10 mM magnesium chloride ($MgCl_2$). The reaction mixture was pre-incubated at 37°C for 5 minutes before adding betanicotinamide adenine dinucleotide phosphate - reduced tetrasodium salt (NADPH) (1.0 mM). Aliquots of the incubation mixture were removed at 0, 5, 10, 20, 30 and 45 minutes. The aliquots were quenched in acetonitrile containing internal standard. Samples were centrifuged and the resulting supernatant analyzed by liquid chromatography/mass spectrometry (LC/MS/MS), with a Sciex API4000 using multiple reaction monitoring optimized for each test compound. The ratios of

the peak area response of test compound to that of the internal standard were used to calculate the rate of disappearance.

The first order disappearance rate constant was determined for each incubation by plotting natural log (LN) substrate concentration (peak area ratio, drug/internal standard) against incubation time and determining the slope of the regression line (k), using data where an accurate first order decay curve could be obtained. Predicted hepatic clearances ($CL_H$) and extraction ratios were calculated according to the following equations:

$$t_{1/2} = \frac{LN(2)}{-k}$$

$$CL'_{int} = \frac{LN(2)}{t1/2\ (min)} \times \frac{21\ g\ liver}{kg\ of\ BW} \times \frac{mL\ incubation}{mg\ of\ microsomal\ protein} \times \frac{45\ mg\ microsomal\ protein}{g\ of\ liver}$$

$$CL_H = \frac{Q_H \cdot CL'_{int}}{Q_H + CL'_{int}}$$

Extraction Ratio (ER) = $CL_H/Q_H$
where the following constants were:

- Liver Weight = 21 g/kg [human]
- Microsomal Protein in Incubation = 1.52 mg/mL
- Liver Protein = 45 mg/g [human]
- Liver Blood Flow (QH) = 20 mUmin/kg [human]

CYP3A Time-Dependent Inhibition: This assay measures the ability of the test compound to inhibit CYP3A in a preincubation time dependent manner. Most marketed macrolides and ketolides are time-dependent inhibitors of CYP3A, a property that can result in drug-drug interactions with CYP3A substrates. This assay measures the potency of the test compound for inhibition of CYP3A ($K_I$) and the rate of inactivation of CYP3A ($k_{inact}$) These 2 parameters can be used in conjunction with the estimated human efficacious concentration of the test compound to predict potential for drug-drug interactions in humans.

Assay Procedure: *Primary incubation:* Pooled human liver microsomes (0.5 μM final CYP concentration) were preincubated in the presence of a NADPH regenerating system [containing β-NADP-Na (0.54 mM), $MgCl_2$ (11.5 mM), dl-isocitric acid (6.2 mM), and isocitric dehydrogenase (0.5 units/mL)] in 100 mM $KH_2PO_4$, pH 7.4, at 37°C open to air in a 0.7 mL total volume. After 5 minutes, aliquots (7 μL) of test compound (100X stocks in DMSO: to yield final concentrations of 0, 1, 5, 10, 50 or 100 μM) were added. Aliquots (36 μL) of the primary incubation mixture were removed at time 0, 5, 10, 20, and 30 minutes, and transferred to secondary incubations, as described below.

*Secondary incubation:* Secondary incubations containing midazolam (30 μMfinal) and NADPH-regenerating system (as described above for primary incubation) were carried out in triplicate in 100 mM $KH_2PO_4$, pH 7.4, at 37 °C open to air in a 0.7 mL total volume. Reactions were initiated by addition of 36 μL primary incubation mixture, and were incubated for a total reaction time of 4 minutes. Reaction velocity had previously been confirmed to be linear for the 4-minute reaction time at a CYP concentration of 0.025 μM. After the 4-minute reaction time, 100 μL of reaction mixture was quenched with 300 μL of ice-cold acetonitrile with internal standard. The acetonitrile solutions were vortexed and centrifuged and the resulting supernatant was assayed for 1'-hydroxymidazolam formation by LC/MS/MS using a Sciex API4000 with multiple reaction monitoring. The ratios of the peak area response of analyte (1'-hydroxymidazolam) to that of the internal standard were used for quantification.

The formation of 1'-hydroxymidazolam as a function of preincubation time and test compound concentration was determined. Data were converted to percent remaining activity by setting the amount of 1'-hydroxymidazolam formed at the zero preincubation time to 100 percent. The natural log (Ln) of the percent remaining activity was plotted for each test compound concentration as a function of preincubation time, and the rate of inactivation (kobs) for each concentration was determined by calculating the slope of the linear portion of the percent remaining activity curve. A plot of kobs versus concentration was then generated, and $K_I$ and $k_{inact}$ parameters were calculated by nonlinear regression of this plot using a 3-parameter hyperbolic fitting program (SigmaPlot v 8.0). Estimation of the effect of time-dependent inhibition

on in vivo CYP3A activity was assessed by incorporating the in vitro $K_I$ and $k_{inact}$ parameters into the following steady state equation, as described by Mayhew et al. Drug Metab Dispos., 28(9):1031-7 (2000).

$$\frac{AUC_{inh}}{AUC} = \frac{k_{deg} + \frac{[I] \times k_{inact}}{[I] + K_I}}{k_{deg}}$$

The terms are defined as described below:

$AUC_{inh}/AUC$ (aka "Predicted Fold Change in AUC) is the ratio of area under the plasma concentration-time curve in the presence of inhibitor ($AUC_{inh}$) and AUC in the absence of inhibitor, for a coadministered drug that is eliminated exclusively by CYP3A metabolism.

$k_{deg}$ represents the estimated in vivo degradation rate constant of CYP3A. A value of 0.0005 $min^{-1}$ ($t_{1/2}$ approximately 23 hours) is used.

$K_{inact}$ represents the maximal rate of inactivation, determined in vitro.

$K_I$ represents the concentration of inhibitor leading to 50% of maximal inactivation, determined in vitro.

[I] represents predicted free steady state average concentration of inhibitor in vivo. The value of [I] is estimated at 0.17 μM, which is the estimated efficacious unbound steady state average concentration for a ketolide.

## Claims

**1.** A compound of the Formula (I);

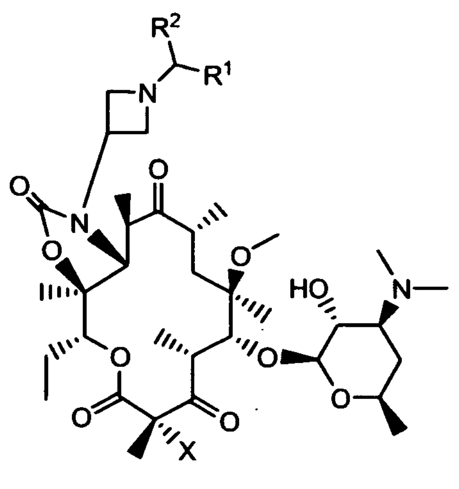

**(I)**

wherein $R^1$ is selected from the group consisting of 3-hydroxy-[1,5]-naphthyridin-4-yl, 3-hydroxy-[1,6]-naphthyridin-4-yl, and 3-hydroxy-[1,7]-naphthyridin-4-yl wherein said 3-hydroxy-[1,5]-naphthyridin-4-yl, 3-hydroxy-[1,6]-naphthyridin-4-yl, and 3-hydroxy-[1,7]-naphthyridin-4-yl are optionally substituted by a group selected from the group consisting of ($C_1$-$C_3$)alkyl, cyclopropyl, and cyclobutyl;

$R^2$ is selected from the group consisting of hydrogen, methyl, and ethyl; and

X is hydrogen or fluorine;

or a pharmaceutically acceptable salt thereof.

**2.** The compound according to claim 1, or a pharmaceutically acceptable salt thereof, wherein $R^1$ is 3-hydroxy-[1,5]-naphthyridin-4-yl.

**3.** The compound according to claim 1, or a pharmaceutically acceptable salt thereof, wherein $R^1$ is 3-hydroxy-[1,6]-naphthyridin-4-yl.

**4.** The compound according to claim 1, or a pharmaceutically acceptable salt thereof, wherein $R^1$ is 3-hydroxy-[1,7]-naphthyridin-4-yl.

**5.** The compound according to claims 1 to 4, or a pharmaceutically acceptable salt thereof, wherein said 3-hydroxy-[1,5]-naphthyridin-4-yl, 3-hydroxy-[1,6]-naphthyridin-4-yl, and 3-hydroxy-[1,7]-naphthyridin-4-yl is substituted by methyl.

**6.** The compound according to claims 1 to 5, or a pharmaceutically acceptable salt thereof, wherein $R^2$ is hydrogen.

**7.** The compound according to claims 1 to 5, or a pharmaceutically acceptable salt thereof, wherein $R^2$ is methyl.

**8.** A compound selected from the group consisting of:

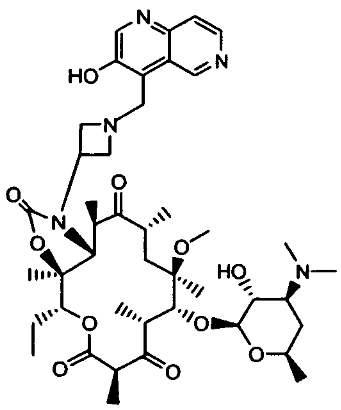
,
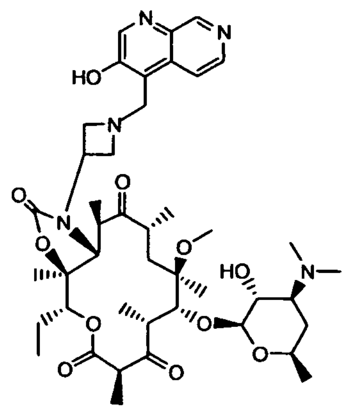
,
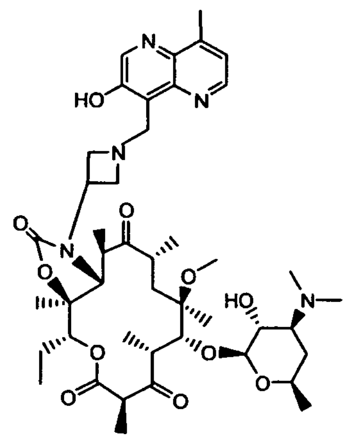
,

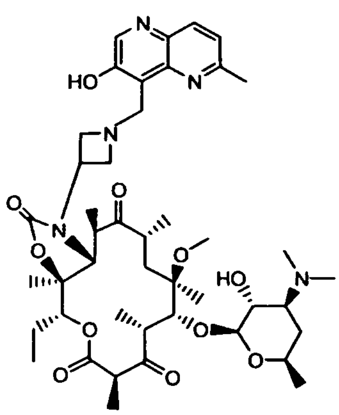
,
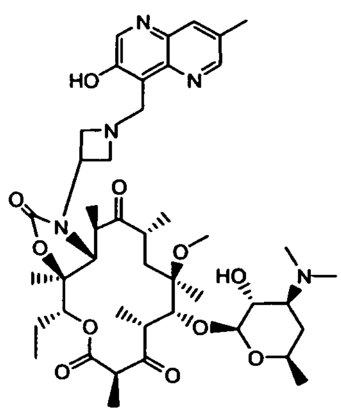
,
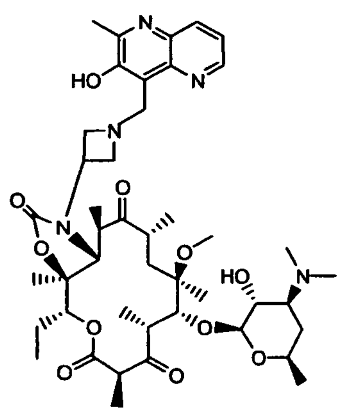
,

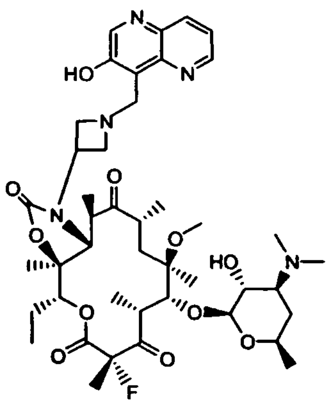 , 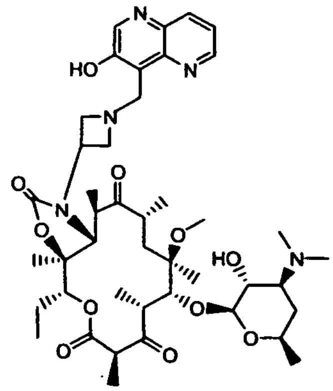 , and 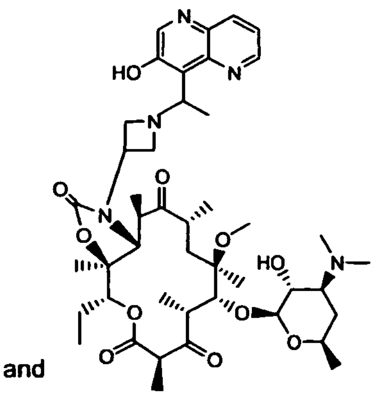 ;

or a pharmaceutically acceptable salt thereof.

**9.** The compound of claim 8, which is

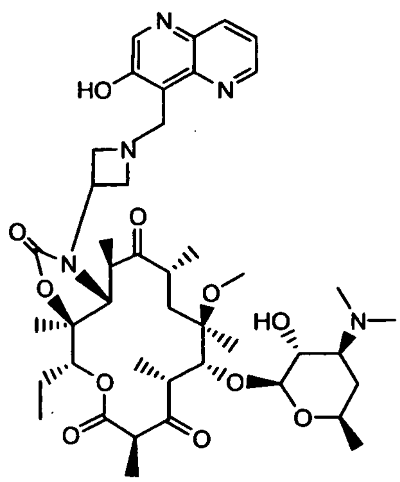

or a pharmaceutically acceptable salt thereof.

**10.** A pharmaceutical composition comprising the compound of claims 1 to 9, or pharmaceutically acceptable salt thereof, and a pharmaceutically acceptable carrier.

**11.** The use of a compound of Formula (I) or pharmaceutically acceptable salt thereof of claims 1 to 9 in the manufacture of a medicament for the treatment of a bacterial infection.

**12.** The use use of compound of formula (I) or pharmaceutically acceptable salt thereof of claims 1 to 9 in the manufacture of a medicament for the treatment of infection caused by at least one of *Streptococcus pyogenes, Streptococcus pneumoniae, Haemophilus influenza,* or *Moraxella catarrhalis.*

**13.** A method of making a compound of Formula (I),

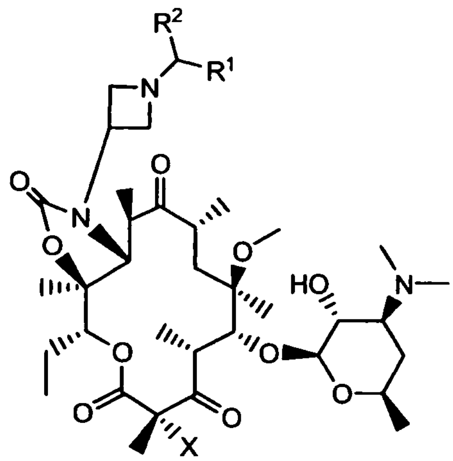

(I)

or pharmaceutically acceptable salt thereof, comprising a first step of reacting a compound of Formula (VIII),

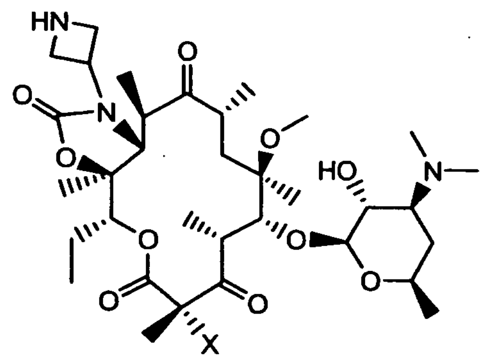

(VIII)

,

with a compound of Formula (IC),

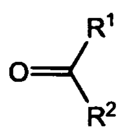

(IC)

in the presence of an acid catalyst and a polar aprotic solvent to form an admixture;
and a second step of treating said admixture with a reducing agent;
wherein $R^1$ is selected from the group consisting of 3-hydroxy-[1,5]-naphthyridin-4-yl, 3-hydroxy-[1,6]-naphthyridin-4-yl, and 3-hydroxy-[1,7]-naphthyridin-4-yl wherein said 3-hydroxy-[1,5]-naphthyridin-4-yl, 3-hydroxy-[1,6]-naphthyridin-4-yl, and 3-hydroxy-[1,7]-naphthyridin-4-yl are optionally substituted by a group selected from the group consisting of $(C_1-C_3)$alkyl, cyclopropyl, and cyclobutyl;
$R^2$ is selected from the group consisting of hydrogen, methyl, and ethyl; and
X is hydrogen or fluorine.

**14.** 3-Descladinosyl-11,12-dideoxy-6-O-methyl-3-oxo-12,11-(oxycarbonyl-(1-((3-hydroxy-[1,5]-naphthyridin-4-yl)-methyl)-azetidin-3-yl)-imino)-erythromycin A fumarate.

**15.** A method of making a compound of Formula (C),

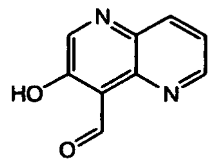

(C)

comprising a first step reacting a compound of Formula (IX),

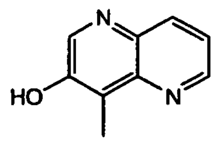

,

(IX)

with a compound of Formula (X),

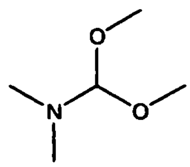

,

(X)

in the presence of a base to make a compound of Formula (XI),

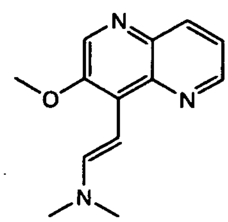

;

(XI)

a second step of reacting a compound of Formula (XI) with sodium periodate in water to form a compound of Formula (XII),

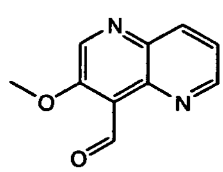

;

(XII)

and a third step of reacting a compound of Formula (XII) in LiCl or HCl to form a compound of Formula (C).

## Patentansprüche

1. Verbindung der Formel (I):

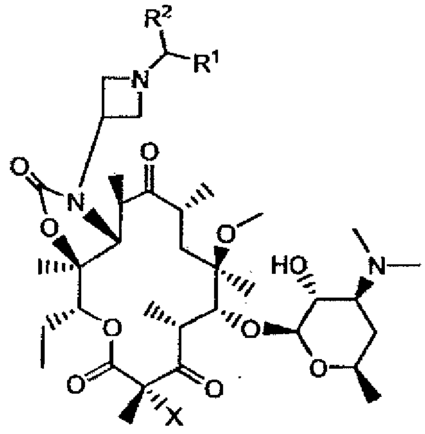

(I)

worin $R^1$ ausgewählt ist aus der Gruppe, bestehend aus 3-Hydroxy-[1,5]-naphthyridin-4-yl, 3-Hydroxy-[1,6]-naphthyridin-4-yl und 3-Hydroxy-[1,7]-naphthyridin-4-yl, wobei die 3-Hydroxy-[1,5]-naphthyridin-4-yl, 3-Hydroxy-[1,6]-naphthyridin-4-yl und 3-Hydroxy-[1,7]-naphthyridin-4-yl gegebenenfalls mit einer Gruppe substituiert sind, die ausgewählt ist aus der Gruppe, bestehend aus $(C_1-C_3)$Alkyl, Cyclopropyl und Cyclobutyl;
$R^2$ ausgewählt ist aus der Gruppe, bestehend aus Wasserstoff, Methyl und Ethyl, und
X Wasserstoff oder Fluor ist,
oder ein pharmazeutisch verträgliches Salz davon.

2. Verbindung nach Anspruch 1 oder ein pharmazeutisch verträgliches Salz davon, wobei $R^1$ 3-Hydroxy-[1,5]-naphthyridin-4-yl ist.

3. Verbindung nach Anspruch 1 oder ein pharmazeutisch verträgliches Salz davon, wobei $R^1$ 3-Hydroxy-[1,6]-naphthyridin-4-yl ist.

4. Verbindung nach Anspruch 1 oder ein pharmazeutisch verträgliches Salz davon, wobei $R^1$ 3-Hydroxy-[1,7]-naphthyridin-4-yl ist.

5. Verbindung nach den Ansprüchen 1 bis 4 oder ein pharmazeutisch verträgliches Salz davon, wobei das 3-Hydroxy-[1,5]-naphthyridin-4-yl, 3-Hydroxy-[1,6]-naphthyridin-4-yl und 3-Hydroxy-[1,7]-naphthyridin-4-yl mit Methyl substituiert ist.

6. Verbindung nach den Ansprüchen 1 bis 5 oder ein pharmazeutisch verträgliches Salz davon, wobei $R^2$ Wasserstoff ist.

7. Verbindung nach den Ansprüchen 1 bis 5 oder ein pharmazeutisch verträgliches Salz davon, wobei $R^2$ Methyl ist.

8. Verbindung, ausgewählt aus der Gruppe, bestehend aus:

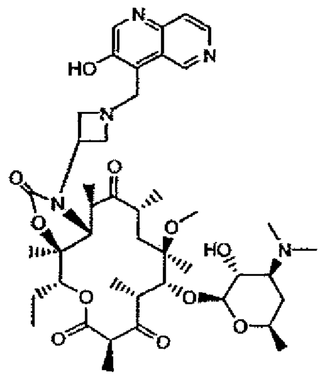, 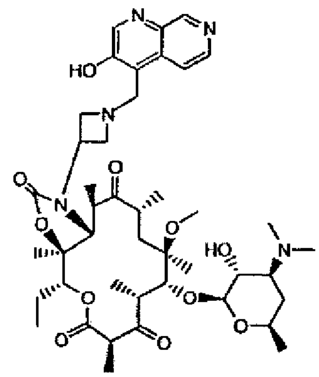, 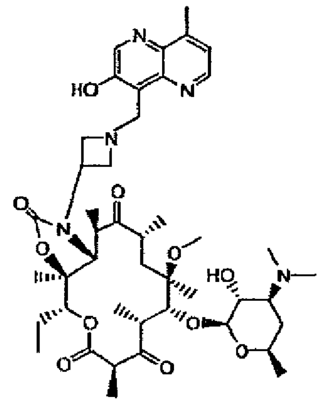.

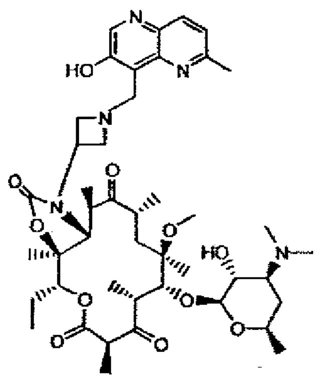, 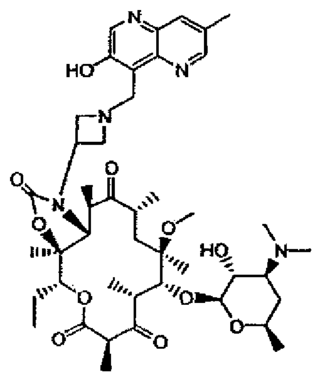, 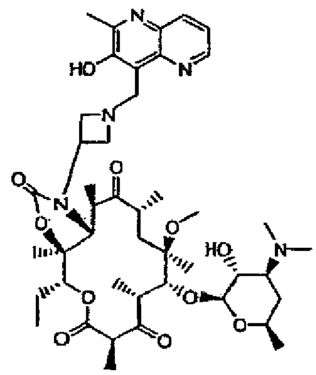.

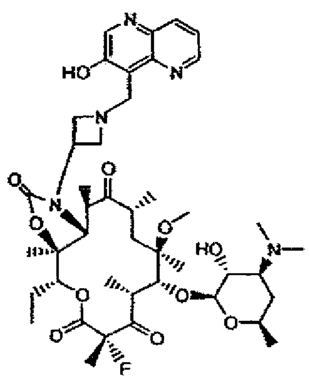, 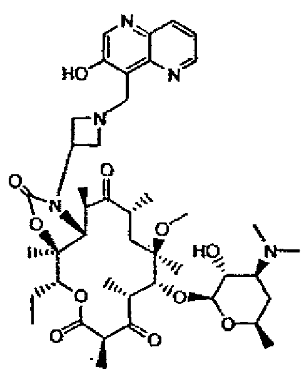 und 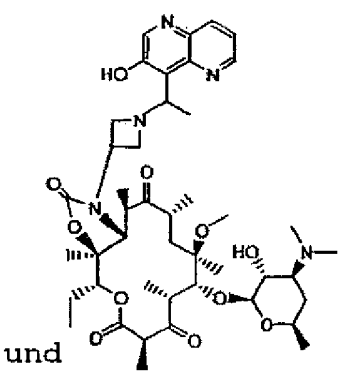

oder ein pharmazeutisch verträgliches Salz davon.

**9.** Verbindung nach Anspruch 8, welche

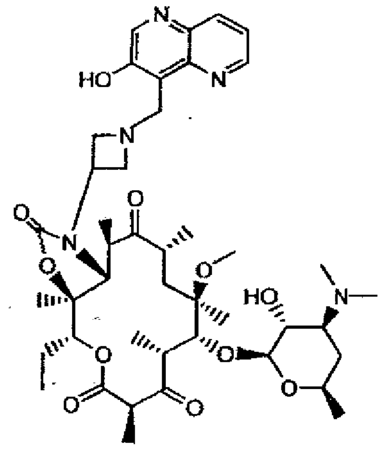

oder ein pharmazeutisch verträgliches Salz davon ist.

**10.** Pharmazeutische Zusammensetzung, umfassend die Verbindung nach den Ansprüchen 1 bis 9 oder ein pharmazeutisch verträgliches Salz davon und einen pharmazeutisch verträglichen Träger.

**11.** Verwendung einer Verbindung der Formel (I) oder eines pharmazeutisch verträglichen Salzes davon nach den Ansprüchen 1 bis 9 bei der Herstellung eines Medikaments für die Behandlung einer bakteriellen Infektion.

**12.** Verwendung einer Verbindung der Formel (I) oder eines pharmazeutisch verträglichen Salzes davon nach den Ansprüchen 1 bis 9 bei der Herstellung eines Medikaments für die Behandlung von Infektion, verursacht durch wenigstens einen von *Streptococcus pyogenes, Streptococcus pneumoniae, Haemophilus influenzae* oder *Moraxella catarrhalis.*

**13.** Verfahren zur Herstellung einer Verbindung der Formel (I)

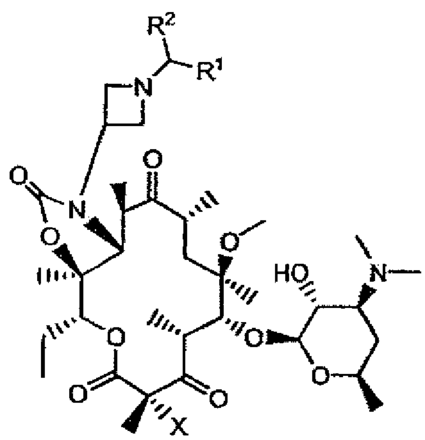

**(I)**

oder eines pharmazeutisch verträglichen Salzes davon, umfassend einen ersten Schritt eines Umsetzens einer Verbindung der Formel (VIII)

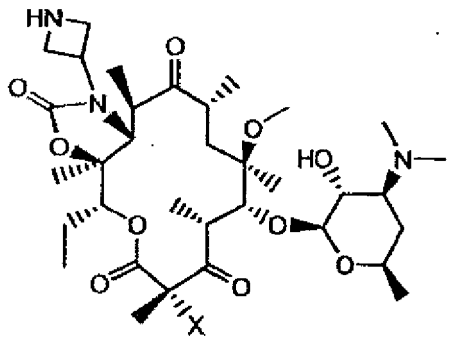

(VIII)

mit einer Verbindung der Formel (IC)

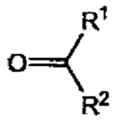

(IC)

in Gegenwart eines Säurekatalysators und eines polaren aprotischen Lösungsmittels unter Bildung eines Gemisches;
und einen zweiten Schritt eines Behandelns des Gemisches mit einem Reduktionsmittel;
wobei $R^1$ ausgewählt ist aus der Gruppe, bestehend aus 3-Hydroxy-[1,5]-naphthyridin-4-yl, 3-Hydroxy-[1,6]-naphthyridin-4-yl und 3-Hydroxy-[1,7]-naphthyridin-4-yl, wobei die 3-Hydroxy-[1,5]-naphthyridin-4-yl, 3-Hydroxy-[1,5]-naphthyridin-4-yl und 3-Hydroxy-[1,7]-naphthyridin-4-yl gegebenenfalls mit einer Gruppe substituiert sind, die ausgewählt ist aus der Gruppe, bestehend aus ($C_1$-$C_3$)Alkyl, Cyclopropyl und Cyclobutyl;
$R^2$ ausgewählt ist aus der Gruppe, bestehend aus Wasserstoff, Methyl und Ethyl, und
X Wasserstoff oder Fluor ist.

**14.** 3-Descladinosyl-11,12-didesoxy-6-O-methyl-3-oxo-12,11-(oxycarbonyl-(1-((3-hydroxy-[1,5]-napthyridin-4-yl)-methyl)-azetidin-3-yl)-imino)-erythromycin A-Fumarat.

**15.** Verfahren zur Herstellung einer Verbindung der Formel (C)

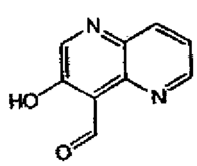

(C)

umfassend einen ersten Schritt eines Umsetzens einer Verbindung der Formel (IX)

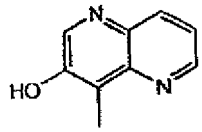

(IX)

mit einer Verbindung der Formel (X)

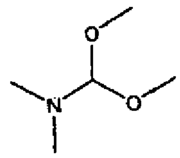

(X)

in Gegenwart einer Base unter Herstellung einer Verbindung der Formel (XI)

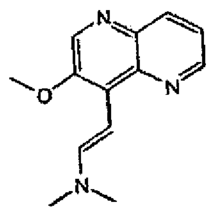

(XI) ;

einen zweiten Schritt eines Umsetzens einer Verbindung der Formel (XI) mit Natriumperiodat in Wasser unter Bildung einer Verbindung der Formel (XII)

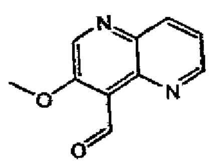

(XII)

und einen dritten Schritt eines Umsetzens einer Verbindung der Formel (XII) in LiCl oder HCl unter Bildung einer Verbindung der Formel (C).

**Revendications**

1. Composé de Formule (I) ;

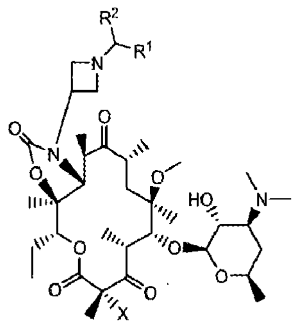

(I)

dans lequel :

$R_1$ est sélectionné dans le groupe consistant en 3-hydroxy-[1,5]-naphtyridin-4-yle, 3-hydroxy-[1,6]-naphtyridin-4-yle et 3-hydroxy-[1,7]-naphtyridin-4-yle, lesdits groupes 3-hydroxy-[1,5]-naphtyridin-4-yle, 3-hydroxy-[1,6]-naphtyridin-4-yle et 3-hydroxy-[1,7]-naphtyridin-4-yle étant facultativement substitués par un groupe sélectionné dans le groupe consistant en alkyle en $C_1$-$C_3$, cyclopropyle et cyclobutyle ;
$R_2$ est sélectionné dans le groupe consistant en l'hydrogène, méthyle et éthyle ; et
X représente l'hydrogène ou le fluor ;

ou sel pharmaceutiquement acceptable dudit composé.

**2.** Composé selon la revendication 1, ou sel pharmaceutiquement acceptable de celui-ci, dans lequel $R_1$ représente 3-hydroxy-[1,5]-naphtyridin-4-yle.

**3.** Composé selon la revendication 1, ou sel pharmaceutiquement acceptable de celui-ci, dans lequel $R_1$ représente 3-hydroxy-[1,6]-naphtyridin-4-yle.

**4.** Composé selon la revendication 1, ou sel pharmaceutiquement acceptable de celui-ci, dans lequel $R_1$ représente 3-hydroxy-[1,7]-naphtyridin-4-yle.

**5.** Composé selon les revendications 1 à 4, ou sel pharmaceutiquement acceptable de celui-ci, dans lequel lesdits groupes 3-hydroxy-[1,5]-naphtyridin-4-yle, 3-hydroxy-[1,6]-naphtyridin-4-yle et 3-hydroxy-[1,7]-naphtyridin-4-yle sont substituée par méthyle.

**6.** Composé selon les revendications 1 à 5, ou sel pharmaceutiquement acceptable de celui-ci, dans lequel $R_2$ représente l'hydrogène.

**7.** Composé selon les revendications 1 à 5, ou sel pharmaceutiquement acceptable de celui-ci, dans lequel $R_2$ représente méthyle.

**8.** Composé sélectionné dans le groupe consistant en :

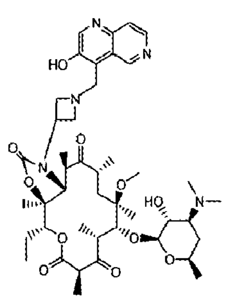 , 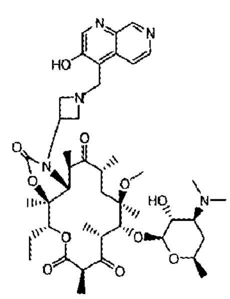 , 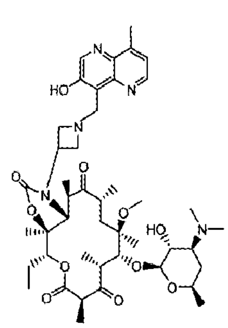 ,

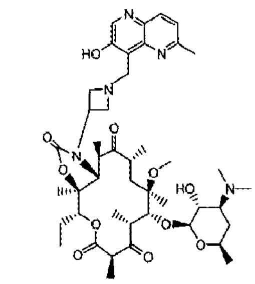
,
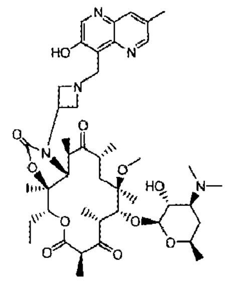
,
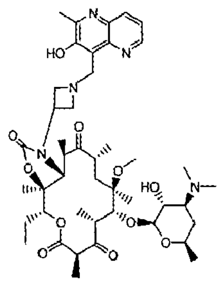
.

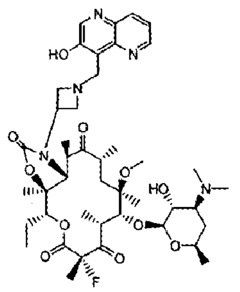
.
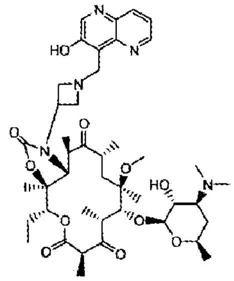
et
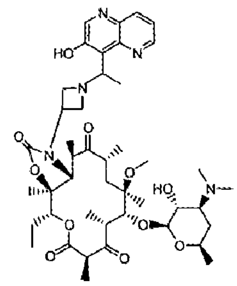
;

ou sel pharmaceutiquement acceptable de celui-ci.

**9.** Composé selon la revendication 8, qui est

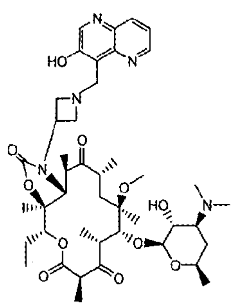

ou sel pharmaceutiquement acceptable de celui-ci.

**10.** Composition pharmaceutique comprenant le composé selon les revendications 1 à 9, ou un sel pharmaceutiquement acceptable de celui-ci, et un support pharmaceutiquement acceptable.

**11.** Utilisation d'un composé de Formule (I) ou d'un sel pharmaceutiquement acceptable de celui-ci, selon les revendications 1 à 9, dans la préparation d'un médicament pour le traitement d'une infection bactérienne.

**12.** Utilisation d'un composé de Formule (I) ou d'un sel pharmaceutiquement acceptable de celui-ci, selon les revendications 1 à 9, dans la préparation d'un médicament pour le traitement d'une infection causée par l'un au moins parmi *Streptococcus pyogenes, Streptococcus pneumoniae, Haemophilus influenzae* ou *Moraxella catarrhalis.*

**13.** Procédé de préparation d'un composé de Formule (I),

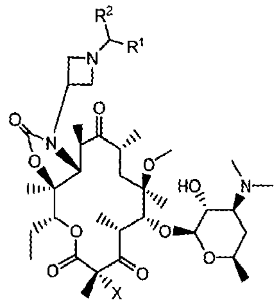

(I)

ou d'un sel pharmaceutiquement acceptable de celui-ci, comprenant une première étape de réaction d'un composé de Formule (VIII),

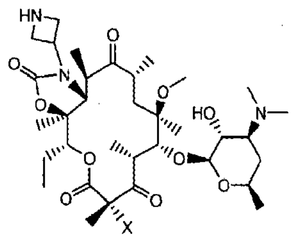

(VIII) ,

avec un composé de Formule (IC),

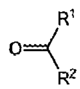

(IC)

en présence d'un catalyseur acide et d'un solvant aprotique polaire pour former un mélange ; et une seconde étape de traitement dudit mélange avec un agent réducteur ;
$R_1$ étant sélectionné dans le groupe consistant en 3-hydroxy-[1,5]-naphtyridin-4-yle, 3-hydroxy-[1,6]-naphtyridin-4-yle et 3-hydroxy-[1,7]-naphtyridin-4-yle, lesdits groupes 3-hydroxy-[1,5]-naphtyridin-4-yle, 3-hydroxy-[1,6]-naphtyridin-4-yle et 3-hydroxy-[1,7]-naphtyridin-4-yle étant facultativement substitués par un groupe sélectionné dans le groupe consistant en alkyle en $C_1$-$C_3$, cyclopropyle et cyclobutyle ;
$R_2$ étant sélectionné dans le groupe consistant en l'hydrogène, méthyle et éthyle ; et
X représentant l'hydrogène ou le fluor.

**14.** Fumarate de 3-descladinosyl-11,12-didéoxy-6-O-méthyl-3-oxo-12,11-(oxycarbonyl-(1-((3-hydroxy-[1,5]-naphtyridin-4-yl)-méthyl)-azétidin-3-yl)-imino)-érythromycine A.

**15.** Procédé de préparation d'un composé de Formule (C),

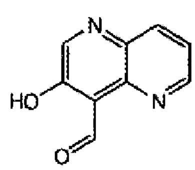

(C)

comprenant une première étape de réaction d'un composé de Formule (IX),

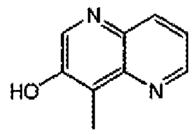

,

(IX)

avec un composé de Formule (X),

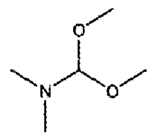

,

(X)

en présence d'une base pour préparer un composé de Formule (XI),

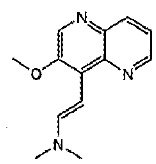

;

(XI)

une seconde étape de réaction d'un composé de Formule (XI) avec du periodate de sodium, dans l'eau, pour former un composé de Formule (XII),

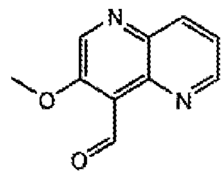

;

**(XII)**

et une troisième étape de réaction d'un composé de Formule (XII) dans du LiCl ou du HCl pour former un composé de Formule (C).

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4331803 A
- US 4474768 A
- US 4517359 A
- US 5523399 A
- US 5527780 A
- US 5635485 A
- US 5804565 A
- US 6020521 A
- US 6025350 A
- US 6075133 A
- US 6162794 A
- US 6191118 B
- US 6248719 B
- US 6291656 B
- US 6437151 B
- US 6472371 B
- US 6555524 B
- US 20020052328 A
- US 20020061856 A
- US 20020061857 A
- US 20020077302 A
- US 20020151507 A
- US 20020156027 A
- US 20030100518 A
- US 20030100742 A
- US 20030199458 A
- US 20040077557 A
- US 20060135447 A
- WO 9911651 A
- WO 9921866 A
- WO 9921869 A
- WO 9935157 A
- EP 1114826 A
- US 2653899 A
- US 2823203 A
- WO 2007026207 A
- WO 2006067589 A


### Non-patent literature cited in the description


- **Tanikawa et al.** *J. Med. Chem.,* 2003, vol. 46, 2706-2715
- **Walsh ; Wright.** *Chemical Reviews,* 2005, vol. 105 (2), 391-394
- **Bush et al.** *Current Opinion in Microbiology,* 2004, vol. 7, 466-476
- **Scaglione et al.** *Journal of Antimicrobial Chemotherapy,* 1998, vol. 41 (B), 47-50
- **Ianaro et al.** *The Journal of Pharmacology and Experimental Therapeutics,* 2000, vol. 292, 156-163
- **Kyburz et al.** *Nature Clinical Practice Rheumatology,* 2006, vol. 2, 458-459
- **K. R. Morris.** Polymorphism in Pharmaceutical Solids. Marcel Dekker, 1995
- **T. W. Greene ; P. G. M. Wuts.** Protective Groups in Organic Synthesis. John Wiley and Sons, 1991
- **L. Fieser ; M. Fieser.** Fieser and Fieser's Reagents for Organic Synthesis. John Wiley and Sons, 1994
- Encyclopedia of Reagents for Organic Synthesis. John Wiley and Sons, 1995
- **Baker et al.** *J. Org. Chem.,* 1988, vol. 53, 2340-2345
- **Mayhew et al.** *Drug Metab Dispos.,* 2000, vol. 28 (9), 1031-7